(19) Europäisches Patentamt
European Patent Office
Office européen des brevets

(11) EP 2 662 382 B1

(12) # FASCICULE DE BREVET EUROPEEN

(45) Date de publication et mention de la délivrance du brevet:
**06.04.2016 Bulletin 2016/14**

(21) Numéro de dépôt: **12305518.8**

(22) Date de dépôt: **10.05.2012**

(51) Int Cl.:
***C07J 9/00*** *(2006.01)* ***C07J 41/00*** *(2006.01)*
***C07J 75/00*** *(2006.01)* ***A61K 31/575*** *(2006.01)*
***A61P 35/00*** *(2006.01)*

(54) **Dérivés de stérol, leur procédé de préparation, les compositions pharmaceutiques les contenant et leur utilisation pour le traitement du glioblastome multiple**

Sterolderivate, ihr Herstellungsverfahren, die sie enthaltenden pharmazeutischen Zusammensetzungen und ihr Einsatz zur Behandlung des Glioblastoms (Glioblastoma multiforme)

Sterol derivatives, method for preparing same, pharmaceutical compositions containing same and use thereof for the treatment of multiple glioblastomas

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**
Etats d'extension désignés:
**BA ME**

(43) Date de publication de la demande:
**13.11.2013 Bulletin 2013/46**

(73) Titulaire: **Beta Innov**
**75015 Paris (FR)**

(72) Inventeurs:
- **Clarion, Ludovic**
**34980 SAINT GELY DU FESC (FR)**
- **Mersel, Marcel**
**34080 MONTPELLIER (FR)**
- **Petite, Didier**
**34000 MONTPELLIER (FR)**

(74) Mandataire: **Santarelli**
**49, avenue des Champs-Elysées**
**75008 Paris (FR)**

(56) Documents cités:
- **WERTHLE MYRIAM ET AL: "Local administration of 7-beta-hydroxycholesteryl-3-oleate inhibits growth of experimental rat C6 glioblastoma", CANCER RESEARCH, AMERICAN ASSOCIATION FOR CANCER RESEARCH, US, vol. 54, no. 4, 15 février 1994 (1994-02-15), pages 998-1003, XP002619892, ISSN: 0008-5472**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

EP 2 662 382 B1

## Description

L'invention concerne de nouveaux dérivés de stérol, leur procédé de préparation, les compositions pharmaceutiques les contenant et leur utilisation pour le traitement de maladies impliquant des cellules astrocytaires transformées en cellules cancéreuses, et en particulier pour le traitement du glioblastome multiple.

La transformation cellulaire correspond au passage d'une cellule eucaryote normale vers une cellule immortalisée et/ou une cellule eucaryote cancéreuse. Dans la suite de la description, on utilisera indifféremment les termes « cellule transformée » ou « cellule cancéreuse ».

Le gtiobtastome multiple (GBM), connu également sous le nom d'astrocynome de grade 4, est une tumeur cérébrale caractérisée par la transformation des cellules astrocytaires en cellules cancéreuses.

Malgré les avancées scientifiques et thérapeutiques substantielles dans le domaine de la cancérologie, le GBM reste un cancer incurable. Au mieux, chercheurs et médecins sont satisfaits lorsque la médiane de vie des patients peut être prolongée de quelques mois, au maximum de quinze mois.

Un des problèmes posés par le traitement du GBM est la rechute causée par les cellules souches. En effet, même lorsque les thérapies existantes parviennent à éradiquer tout ou partie de la tumeur, les cellules souches (en anglais « stem cells ») sont souvent à l'origine d'un nouveau départ tumoral (1,2).

Les thérapies actuelles consistent toujours en une résection de la tumeur, si sa localisation le permet, suivie d'une radiothérapie et/ou d'une chimiothérapie selon le cas. En chimiothérapie, un des traitements « leader » est une bithérapie qui consiste à administrer de l'avastin (inhibition de l'association du VEGF à ses récepteurs) et de l'irinotecan (inhibiteur de la topoisomérase I). La trithérapie de type PCV, qui est une combinaison de Procarbazine (agent alkylant de l'ADN), de lomustine (CCNU ; agent alkylant non spécifique) et de vincristine (inhibition de la polymérisation des microtubules) est à l'heure actuelle très controversée. Le temozolomide, agent alkylant de la guanine, en association avec une radiothérapie montre une augmentation de la médiane de survie en particulier pour les patients qui ont un ADN hyperméthylé. Des essais cliniques (phase III) qui testent le cilengitide (inhibition de quelques récepteurs à l'intégrine) et le talampanel (blocage des canaux au glutamate de type AMPA) sont en cours.

Les recherches et essais cliniques en immunothérapie sont de dieux types:

- l'immunothérapie adaptative, dans laquelle des cellules activées in vitro sont injectées chez le patient tels des lymphocytes tueurs activées (LAK; essais cliniques phase II) ou des lymphocytes cytotoxiques (CTL ; essais cliniques de phase I) injectés en mode intracrânien. A l'heure actuelle, les observations montrent une survie de 20 mois de façon très marginale.
- l'immunothérapie active, qui consiste en l'utilisation de vaccins (phase I) et de cellules dendritiques (phase II). Celle-ci ne montre pas d'amélioration significative de la survie des patients. Ces essais ont été stoppés.

Les thérapies géniques qui consistent en l'utilisation d'adénovirus, de rétrovirus ou de virus de la rougeole comme vecteur de molécules à potentiel anticancéreux montrent une amélioration de la survie de 6 à 11 mois seulement. La thérapie cellulaire qui propose d'utiliser des cellules souches neuronales comme transporteurs de médicaments dans les GBM est encore au stade de la démonstration en recherche fondamentale.

Une approche un peu délaissée ces dernières décennies, et qui est à nouveau envisagée, consiste à agir au niveau de la glycolyse et de la phosphorylation oxydative. Les cellules cancéreuses augmentent leur consommation de glucose du fait qu'elles tendent à basculer leur métabolisme vers un métabolisme anaérobique, même si l'apport en oxygène n'est pas un facteur limitant. Ce phénomène, observé par Warburg (3) est dû à une surexpression de HIF (Hypoxia Induced Factor) et du pro-oncogène Myc. HIF augmente la conversion de pyruvate en lactate, par voie anaérobique, en inactivant la pyruvate déshydrogénase, qui est une enzyme clé dans la respiration aérobie. Myc stimule la biosynthèse de la glutamine, elle-même impliquée dans la respiration anaérobique (4).

Dans ce contexte, des essais cliniques agissant sur le métabolisme énergétique sont en cours. On peut citer, à titre d'exemple (4) :

- la metformine : inhibiteur du complexe I de la respiration mitochondriale qui à son tour induit l'AMPK qui ralentit la prolifération cellulaire;
- la phorétine : réducteur de l'import du glucose ;
- le phénylacétate : réducteur du taux de glutamine;
- le dichloroacétate : inhibiteur de la pyruvate déshydrogénase.

Toutes ces molécules, excepté le dichloroacétate, sont testées (essais cliniques phase II) et les observations ne sont pas encore connues à l'heure actuelle.

On a maintenant trouvé que des dérivés de stérol ciblant une spécificité du métabolisme énergétique de l'astrocyte, type cellulaire à l'origine des GBM, pouvaient être utilisées pour traiter le glioblastome multiple.

L'originalité de l'invention consiste à utiliser le métabolisme énergétique particulier de la cellule astrocytaire (d'origine gliale) dont la transformation *in fine* mène à la formation des GBM.

En effet, la cellule astrocytaire se sert en même temps de l'apport d'énergie, sous forme d'ATP, par le biais de la phosphorylation oxydative (cycle de Krebs couplé au transport d'électrons dans la mitochondrie) et par le biais du cycle de la glycolyse : dans ce dernier, le pyruvate n'entre pas dans le cycle de Krebs mais est réduit en lactate par l'enzyme lactate déshydrogénase (LDH) de type 5. En plus de l'apport en ATP, l'astrocyte se sert de la glycolyse, via la production de lactate, pour suppléer en neurotransmetteur (glutamate) la cellule voisine, le neurone.

Dans le tableau ci-dessous, le métabolisme énergétique de l'astrocyte, normal ou cancéreux, est comparé de manière schématique avec celui d'autres cellules:

| | Astrocytes | | Autres cellules | |
|---|---|---|---|---|
| | Respiration mitochondriale | Glycolyse | Respiration mitochondriale | Glycolyse |
| Cellules normales | 50% | 50% | 90% | 10% |
| Cellules cancéreuse | **GBM 1%** | **GBM 99%** | 1% | 99% |

Le métabolisme énergétique de l'astrocyte est particulier : en effet, la respiration mitochondriale et la glycolyse fonctionnent en duo.

C'est sur cette dualité métabolique spécifique de la cellule astrocytaire normale, à savoir la respiration mitochondriale, d'une part, et la glycolyse, d'autre part, que repose la stratégie de préparation des dérivés de stérol selon l'invention.

En effet, les inventeurs ont fait l'hypothèse de travail selon laquelle lés dérivés de stérol selon l'invention peuvent orienter le métabolisme énergétique des cellules cancéreuses astrocytaires de la glycolyse vers la respiration mitochondriale, processus qui entraînerait leur mort.

Le 7β-hydroxycholestérol (7β-OHCH), molécule à fort potentiel anticancéreux (5,6), montre une cytoxicité remarquable sur des lignées astrocytaires immortalisées (spontanément transformées) (7,8)de rat et desGBM (lignée C6 de rat) « in vitro » (9). Des études démontrent que l'estérification du 7β-OHCH en C3-OH par les acides gras intracellulaires (formation de 7β-OHCH-C3-ester) était fortement impliquée dans l'effet toxique de la molécule mère, le 7β-OHCH (7, 8, 10).

Cependant le mécanisme d'action du 7β-OHCH, estérifié ou non en C3-OH, sur les GBM « in vitro » était loin d'être élucidé. Récemment, des travaux, effectués sur les lignées C6 ont montré que le 7β-OHCH module l'architecture et la dynamique des rafts, micro-domaines de la membrane plasmique, sièges de l'initiation de certaines messageries cellulaires dont celle de la protéine kinase Akt, enzyme-clé du métabolisme énergétique cellulaire (11). En effet, l'oxystérol, en perturbant l'architecture des rafts, toucherait, en conséquence, l'activité de l'Akt, particulièrement lors de la transformation des cellules normales en cellules cancéreuses : L'Akt régule la capture du glucose et l'activité de la glycolyse dans ces cellules.

De manière surprenante, on a maintenant trouvé que les dérivés de stérol selon l'invention, ayant une structure de base 7beta-hydroxycholestérol portant des substituants en position 3 et des groupements protecteurs en position 7, permettaient simultanément l'inhibition de la glycolyse, essentielle à l'apport d'énergie de l'astrocyte cancéreux de haut grade et, en même temps, de relancer la respiration mitochondriale, elle-même aussi « mortelle » pour cette cellule.

En effet, cette double action conduit à une « surchauffe » de la cellule cancéreuse, entraînant sa mort.

De plus, on a pu montrer que les dérivés de stérol selon l'invention ont également une activité vis-à-vis des cellules souches, permettant ainsi la destruction totale des cellules de glioblastome.

L'invention concerne donc des composés de formule (I)

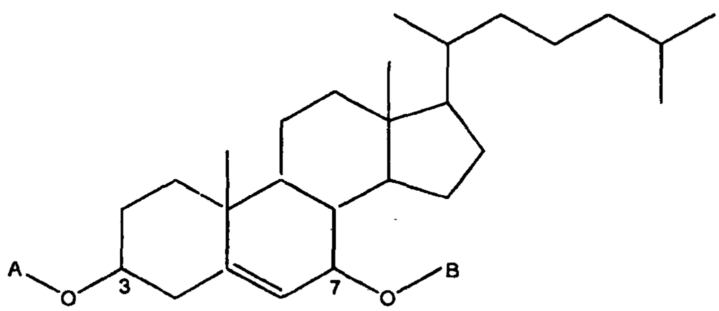

(I)

dans laquelle

- A représente
un groupement $-(R_1)_n-$ dans lequel $R_1$ est un reste d'acide aminé lié par son extrémité C-terminale et n= 1 à 3, chacun des $R_1$ étant identique ou différent, dans lequel l'extrémité N-terminale dudit acide aminé peut être substituée par un groupe $-C-(O)-R_2$ dans lequel $R_2$ est un groupe arylalkyl mono-ou pluri-cyclique en $C_6$-$C_{14}$; un groupe hétéroarylalkyl mono-ou pluri-cyclique en $C_5$-$C_{14}$ pouvant comporter un ou plusieurs hérétoatomes, identiques ou différents; un groupe arylalkyloxy mono-ou pluri-cyclique en $C_6$-$C_{14}$ ou un groupe hétéroarylalkyloxy mono-ou piuri-cydique en $C_5$-$C_{14}$ pouvant comporter un ou plusieurs hérétoatomes, identiques ou différents,
un groupement $-C(O)-NH-R_3$ ou $-C(S)-NH-R_3$ dans lequel $R_3$ est l'hydrogène; un groupe alkyle en $C_1$-$C_{12}$, linéaire ou ramifié, non substitué ou substitué par un groupement choisi parmi OR, NRR', NHR et SR, où est R et R', indépendamment, représentent l'hydrogène, un alkyle linéaire en $C_1$-$C_{12}$ ou un aryle non substitué; un groupe aryle, non substitué ou substitué par un groupement choisi parmi OR, NRR', NHR et SR, tels que définis ci-dessus; un groupe acyle ; un groupe formyle ; un groupe sulfonyle; un groupe sulfinyle; un groupe silyle non substitué ou substitué par un groupe aryle mono- ou pluri-cyclique en $C_6$-$C_{14}$, non substitué ou substitué par au moins un alkyle en $C_1$-$C_6$ linéaire ou ramifié; un groupe allyle ; un reste de sucre ; un groupe $-C(O)-OR_4$ dans lequel $R_4$ est un alkyle en $C_1$-$C_{12}$, linéaire ou ramifié, non substitué ou substitué par un groupement choisi parmi OR, NRR', NHR et SR, tels que définis ci-dessus; un groupe amide ; un groupe thioamide ; un groupe sulfonamide,
un groupement $-C(O)-R_5$ dans lequel $R_5$ est un hétérocycle saturé en $C_5$-$C_{14}$ comprenant 1 ou 2 hétéroatomes, non substitué ou substitué par au moins un alkyle en $C_1$-$C_6$ linéaire ou ramifié ou un groupement choisi parmi OR, NRR', NHR et SR, où est R et R', indépendamment, représentent l'hydrogène, un alkyle en $C_1$-$C_{12}$ ou un aryle non substitué.
- B représente un groupement $-C(O)-R_6$, dans lequel $R_6$ est l'hydrogène; un alkyle en $C_1$-$C_{12}$, de préférence en $C_1$-$C_6$, linéaire ou ramifié, non substitué ou substitué par un groupement choisi parmi OR, NRR', NHR et SR, tels que définis ci-dessus; un groupe aryle, non substitué ou substitué par un groupement choisi parmi OR, NRR', NHR et SR, tels que définis ci-dessus; ou encore $R_6$ représente $OR_7$, dans lequel $R_7$ est un alkyle en $C_1$-$C_{12}$, de préférence en $C_1$-$C_6$, linéaire ou ramifié.

Le groupement alkyle désigne un groupement linéaire ou ramifié en $C_1$-$C_{12}$ tel que les groupements méthyle, éthyle, propyle, isopropyle, butyle, isobutyle, sec-butyle, tert-butyle, pentyle, isopentyle, sec-pentyle, tert-pentyle, néo-pentyle, hexyle, isohexyle, sec-hexyle, tert-hexyle, heptyle, octyle, nonyle, décyle, undécyle ou dodécyle, les groupements alkyles linéaires ou ramifiés en $C_1$-$C_6$ étant préférés.
Le groupement aryle désigne un groupement insaturé, monocyclique ou pluricyclique, carbocyclique, en $C_6$-$C_{14}$, tel que les groupements phényle, naphtyle, indényle, anthracényle et plus particulièrement le groupement phényle.
Par « hétérotaome », on entend un atome d'oxygène, d'azote ou de soufre.
Par « reste de sucre », on entend, par exemple, un motif de type glucose, ribose ou arabinose.
Des restes d'acides aminés avantageux sont, par exemple, des motifs méthionyl, glycinyl ou alanyl.
Des composés préférés de formule (I) sont ceux dans lesquels au moins une des conditions suivantes est remplie :

- A représente un groupement $-(R_1)_n-$ dans lequel $R_1$ est un reste d'acide aminé et n = 2 ;
- A représente un groupement $-(R_1)_n-$ dans lequel $R_1$ est un reste d'acide aminé, n = 2 et l'extrémité N-terminale dudit acide aminé est substituée par un groupe arylalcoxycarbonyl, en particulier benzyloxycarbonyl ;
- A représente un radical alanyl lié à un radical glycinyl , éventuellement substitué sur son extrémité N-terminale par un groupe arylalcoxycarbonyl, en particulier benzyloxycarbonyl;
- A représente un radical méthionyl lié à un radical glycinyl, éventuellement substitué sur son extrémité N-terminale par un groupe arylalcoxycarbonyl, en particulier benzyloxycarbonyl ;
- A représente un groupement $-C(O)-R_5$ dans lequel $R_5$ est un groupe 2,2 diméthyl-1,3-dioxolane.

Avantageusement, B représente un groupement acyl dans lequel le groupe alkyl est en $C_1$-$C_6$, en particulier acétyl ou encore un groupement alcoxycarbonyl dans lequel le groupe alkyl est en $C_1$-$C_6$, en particulier un groupement teit-butoxycarbonyl,
**[0032]** Des composés préférés de formule (I) sont les suivants :

- le 7-((tert-butoxycarbonyl)oxyl)-10,13-diméthyl-17-(6-méthylheptan-2-yl)-2,3,4,7,8,9,10,11,12,13,14,15,16,17-tétradécahydro-1*H*-cyclopenta[*a*]phenanthren-3-yl 2-(2-(((benzyloxy)carbonyl)amino)-acétamido)propanoate (molécule 1.a) ;
- le 7-acétoxy-10,13-diméthyl-17-(6-méthylheptan-2-yl)-2,3,4,7,8,9,10,11,12,13,14,15,16,17-tétradécahydro-1H-cyclopenta[a]phenanthren-3-yl 2-(2-(((benzyloxy)carbonyl)amino)-acétamido)propanoate (molécule 1.b) ;

- le 7-((tert-butoxycarbonyl)oxy)-10,13-diméthyl-17-(6-méthylheptan-2-yl)-2,3,4,7,8,9,10,11,12,13,14,15,16,17-tétradécahydro-1H-cyclopenta[a]phenanthren-3-yl 2,2-diméthyl-1,3-dioxolane-4-carboxylate (molécule 2.a) ;
- le 7-acétoxy-10,13-diméthyl-17-(6-méthylheptan-2-yl)-2,3,4,7,8,9,10,11,12,13,14,15,16,17-tétradécahydro-1H-cyclopenta[a]phenanthren-3-yl 2,2-diméthyl-1,3-dioxolane-4-carboxylate (molécule 2.b).

Les composés de formule (I) peuvent être obtenus à partir du cholestérol par un procédé comprenant les étapes suivantes :

- protection de la fonction hydroxyle en position 3 par un groupement protecteur, tel que, par exemple, un groupement arylalcoxycarbonyl,
- introduction d'une fonction cétone en position 7,
- réduction de la fonction cétone en fonction hydroxyle,
- introduction d'un groupement protecteur sur la fonction hydroxyle en position 7, correspondant au groupement B, tel que par exemple un groupement acyl ou alcoxycarbonyl,
- déprotection de la fonction hydroxyle en position 3.

Après déprotection de la fonction hydroxyle en position 3, ladite fonction hydroxyle peut être substituée par le groupement A souhaité.

L'invention concerne également les compositions pharmaceutiques ou médicaments comprenant au moins un composé de formule (I) et un véhicule pharmaceutiquement acceptable.

La composition pharmaceutique selon l'invention, peut consister en une préparation liposomale comprenant au moins un composé de formule (I). Les liposomes peuvent être fabriqués à l'aide de différentes techniques connues de l'homme du métier. Différents lipides constituant les liposomes peuvent être utilisés [Medical Application of Liposomes (1986) edited by Kunio Yagi, Japan Scientific Societies Press, Tokyo, Karger].

Un médicament préféré de l'invention consiste à cet égard en un liposome chargé en au moins un composé de formule (I).

De préférence, le(s) composé(s) de formule (I) constituent le(s) seul(s) principe(s) actif(s) contenu(s) dans la composition pharmaceutique selon l'invention, notamment lorsque ladite composition pharmaceutique est un liposome.

Alternativement, le composé de formule (I) peut être utilisé en association avec un autre principe actif, tel que, par exemple un agent anticancéreux, en particulier l'avastin, l'irinotécan, le témozolomide ou les dérivés du taxol.

Les compositions pharmaceutiques selon l'invention décrites plus haut peuvent prendre toute forme appropriée pour leur administration orale ou parentérale, en particulier par injection, perfusion ou inhalation, connue de l'homme du métier.

En particulier, ladite composition pharmaceutique peut être une solution pharmaceutiquement acceptable, en particulier une solution alcoolique, d'au moins un composé de formule (I), seul ou en association avec un autre principe actif, qui peut être administrée à un patient par transfusion ou perfusion.

Les composés de formule (I), pour leur utilisation dans le traitement de maladies impliquant des cellules astrocytaires transformées, en particulier le traitement du glioblastome multiple (GBM), sont également un objet de l'invention. En particulier, les composés de formule (I) peuvent être injectés directement dans le cortex cérébral, au site de traitement.

L'invention concerne également le traitement de maladies impliquant des cellules astrocytaires transformées, en particulier le traitement du glioblastome multiple (GBM), par administration d'une quantité efficace d'au moins un composé de formule (I).

Selon une alternative, ledit traitement est un traitement séquentiel qui comprend au moins une étape d'administration d'un premier composé de formule (I) et au moins une étape d'administration d'un deuxième composé de formule (I), différent du premier.

Les exemples suivants illustrent l'invention de manière non limitative.

La partie I concerne la synthèse chimique.

Les exemples 1 et 2 concernent la préparation de composés intermédiaires de synthèse utilisés pour la préparation des composés de formule (I). Les exemples 3 à 6 concernent la préparation de composés de formule (I).

La partie II concerne l'activité biologique des composés de formule (I).

**I/ Synthèse chimique**

Exemple 1 : préparation du 7beta-acétylcholestérol (composé 1.4)

Le schéma réactionnel est représenté sur la Figure 1.

1) Préparation du composé 1.1

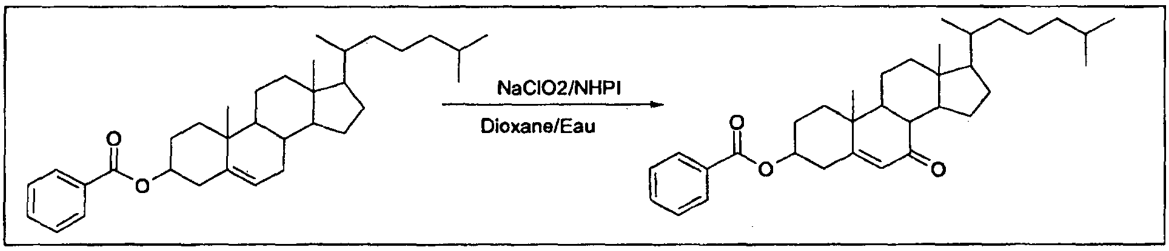


Les réactifs suivants ont été utilisés :

| | MW | Nb mol. | éq | Masse ou Volume |
|---|---|---|---|---|
| Cholestéryl benzoate | 490,78 | 100 mmol | - | 50g |
| Sodium Chlorite | 90,44 | 300 mmol | 3éq | 28g |
| N-hydroxyphtalimide | 163,13 | 10 mmol | 0,1éq | 1,7g |
| Dioxane/Eau 3/1 | | | | 500ml |

Dans un tricol de 1l surmonté d'un réfrigérant, sont placés dans l'ordre le cholesteryl benzoate, le mélange dioxane/eau, le chlorite de sodium et le N-hydroxyphtalimide. Ce mélange est chauffé à 50°C durant 6h. Le suivi de l'avancement de la réaction est réalisé par CCM sur plaque de silice (TLC silica gel 60 F254, Merck) dans Hexane/Et2O 8/2.

Lorsque le taux de formation atteint une valeur acceptable, le brut réactionnel est coulé sur une solution à 10% de sulfite de sodium (500ml), puis extrait à l'éther. La phase organique obtenue est lavé par une solution saturée d'hydrogénocarbonate de sodium, puis à l'eau et enfin à la saumure. Cette phase organique est alors séchée sur sulfate de sodium, filtrées puis évaporées sous pression réduite.

Le résidu huileux coloré obtenu est alors purifié par recristallisation dans l'éthanol. Le solide blanc obtenu n'ayant pas une pureté suffisante, il est repurifié par chromatographie sur gel de silice (silice SDS 60A, 35-70μm). On réalise un dépôt solide en reprenant cette huile dans le dichlorométhane. La purification est réalisée dans un éluant allant de 98/2 Hexane/Acétate d'éthyle à 90/10. Le produit est obtenu sous forme d'un solide blanc.

**Rendement :** 23 %

**Analyses :** Analyse par RMN $^1H$ dans le $CDCl_3$, BRUKER 400 MHz. HPLC colonne phase normale CHIRALCEL O-DH (colonne ODHOCE-CE026), éluant 9/1 Hexane/iPrOH, 20min, longueur d'onde 190nm.
Temps de rétention 6,682 mn, pureté HPLC 99,2%.

**1H RMN (CDCl3, 400,13 MHz) :** δ 0,71 (s, 3H, CH3), 0,89 (dd, 6H, 2CH3), 0,94 (d, 3H, CH3), 1,02-2,78 (m, 26H), 1,27 (s, 3H, CH3), 4,99 (m, 1H, CH), 5,76 (d, 1H, CH), 7,44-8,05 (m, 5H, CHAr).

2) Préparation du composé 1.2

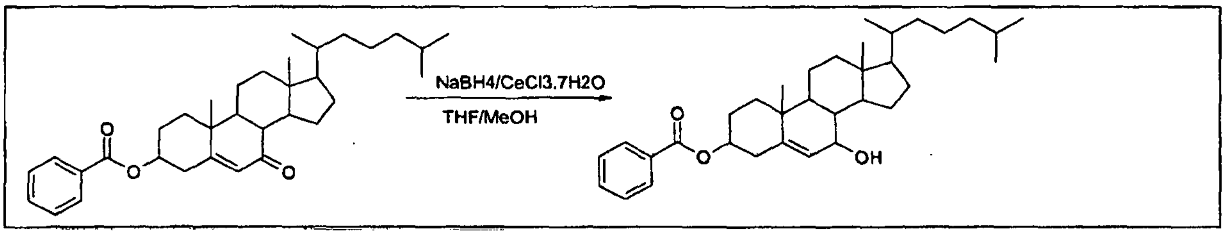

Les réactifs suivants ont été utilisés :

| | MW | Nb mol. | éq | Masse ou Volume |
|---|---|---|---|---|
| Cétocholestéryl benzoate | 504,76 | 15,8 mmol | - | 8 g |
| $NaBH_4$ | 37,83 | 7,9 mmol | 0,5 éq + 0,5 éq | 0,3 g + 0,3 g |
| $CeCl_3$.7H2O | 372,60 | 14,2 mmol | 0,9 éq | 5,3 g |
| THF/MeOH 1:1 | | | | 200 ml |

Dans un ballon de 500ml, on place le cétocholestéryl benzoate, le mélange de solvants THF/MeOH et le chlorure de cérium hydraté. Le brut est alors refroidi à 0°C à l'aide d'un bain de glace, avant d'ajouter lentement le borohydrure de sodium. On observe un dégagement gazeux, le bain de glace est maintenu 1 h, puis on agite à température ambiante pour 18h. On effectue le suivi de l'avancement par CCM (TLC silica gel 60 F254, Merck) dans un éluant 80/20 Hexane/AcOEt. Si le taux de formation n'est pas suffisant on ajoute 0,5éq de borohydrure de sodium.

On ajoute au brut réactionnel 50ml d'eau et 200ml de dichlorométhane. Après transfert en ampoule à décanter, on récupère la phase organique. La phase aqueuse est réextraite au DCM. Les phases organiques sont rassemblées, lavées par une solution d'acide chlorhydrique 1 N puis une solution saturée en NaCl.

La phase organique est alors séchée sur sulfate de sodium, filtrée et évaporée sous pression réduite pour conduire à une huile faiblement colorée qui cristallise spontanément.

On réalise un dépôt solide en reprenant le résidu dans du DCM. On purifie ce brut sur colonne de gel de silice (silice SDS 60A, 35-70$\mu$m) dans un éluant Hexane/AcOEt 9/1. Le produit est obtenu sous forme d'un solide blanc.

**Rendement :** 89%

**Analyse :** Analyse par RMN $^1$H dans le $CDCl_3$, BRUKER 400 MHz. HPLC colonne phase normale CHIRALCEL O-DH (colonne ODHOCE-CE026), éluant 9/1 Hexane/iPrOH, 20mn, longueur d'onde 190nm.

Temps de rétention 7,076 mn, pureté HPLC 98,8%.

**1H RMN (CDCl3, 400,13 MHz):** $\delta$ 0,63 (s, 3H, CH3), 0,79 (dd, 6H, 2CH3), 0,85 (d, 3H, CH3), 0,89-2,43 (m, 27H), 1,04 (s, 3H, CH3), 3,81 (d, 1H, CH), 4,81 (m, 1 H, CH), 5,29 (d, 1 H, CH), 7,34-7,99 (m, 5H, CHAr).

3) Préparation du composé 1.3

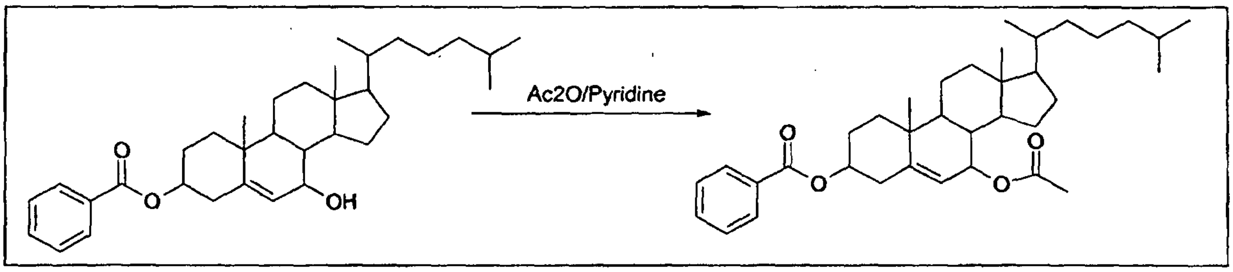


Les réactifs suivants ont été utilisés :

| | MW | Nb mol. | éq | Masse ou Volume |
|---|---|---|---|---|
| 7-$\beta$-hydroxycholestéryl benzoate | 505,76 | 9,9 mmol | - | 5 g |
| Anhydride acétique | | | | 20 ml |
| Pyridine | | | | 20 ml |

Dans un ballon de 100ml, on place le 7-$\beta$-hydroxycholestéryl benzoate, la pyridine, puis l'anhydride acétique. Ce mélange est agité à température ambiante pour 16h. Le suivi de l'avancement est réalisé par CCM (TLC silica gel 60 F254, Merck) dans un éluant 9/1 Hexane/AcOEt.

Le brut réactionnel est évaporé sous pression réduite. On effectue deux coévaporations à l'acétate d'éthyle. Le résidu obtenu est repris dans l'acétate d'éthyle. La phase organique ainsi obtenue est lavée à l'acide chlorhydrique

1 N, séchée sur sulfate de sodium puis évaporée sous pression réduite.

Le solide blanc obtenu est directement introduit dans l'étape suivante sans purification supplémentaire.

**Rendement:** 100%

**Analyse :** Analyse par RMN $^1H$ dans le $CDCl_3$, BRUKER 400 MHz. HPLC colonne phase normale CHIRALCEL O-DH (colonne ODHOCE-CE026), éluant 9/1 Hexane/iPrOH, 20mn, longueur d'onde 190nm.

Temps de rétention 4,972 mn, pureté HPLC 99,6%.

**1H RMN (CDCl3, 400,13 MHz)** : δ 0,63 (s, 3H, CH3), 0,79 (dd, 6H, 2CH3), 0,84 (d, 3H, CH3), 0,91-2,41 (m, 26H), 1,06 (s, 3H, CH3), 1,95 (s, 3H, CH3 acétyle), 4,78 (m, 1H, CH), 4,99 (d, 1H, CH), 5,21 (s, 1H, CH), 7,33-7,98 (m, 5H, CHAr).

4) Préparation du composé 1.4

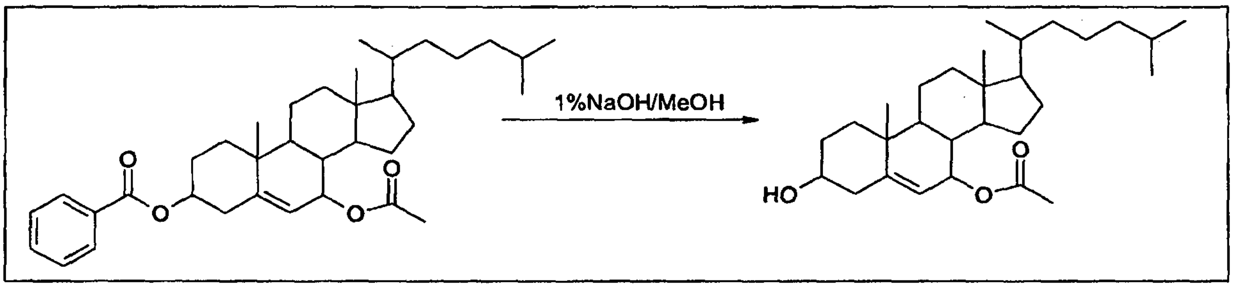


Les réactifs suivants ont été utilisés :

| | MW | Nb mol. | éq | Masse ou Volume |
|---|---|---|---|---|
| 7-β-acétylcholestéryl benzoate | 548,81 | 9,1 mmol | - | 5g |
| 1 % NaOH dans Méthanol | | | | 50 ml |

Dans un ballon de 100ml, on place le 7-β-acétylcholestéryl benzoate et la solution 1% de soude dans le méthanol. Ce mélange est agité à température ambiante jusqu'à complète solubilisation. Le suivi de l'avancement est réalisé par CCM (TLC silica gel 60 F254, Merck) dans un éluant 7/3 Hexane/AcOEt.

Afin de compléter la réaction le brut peut être chauffé à 40°C, un contrôle par CCM est alors réalisé toutes les 20 minutes.

On ajoute 200 ml d'acétate d'éthyle et 50ml d'eau, puis on transfère en ampoule à décanter et séparation des phases. La phase aqueuse est réextraite à l'acétate d'éthyle. Les phases organiques sont rassemblées, séchées sur sulfate de sodium, filtrées puis évaporées sous pression réduite pour conduire à un résidu huileux.

Le résidu est repris dans l'acétate d'éthyle afin de préparer le dépôt solide. La purification est réalisée sur colonne de gel de silice (silice SDS 60A, 35-70μm) dans un éluant Hexane/AcOEt allant de 9/1 à 7/3. Le produit attendu est obtenu sous forme d'une huile incolore qui cristallise spontanément pour conduire à un solide blanc. La colonne est lavée à l'acétate d'éthyle 100% afin de récupérer le 7-β-hydroxycholestérol formé.

**Rendement :** 38%

**Analyse :** Analyse par RMN $^1H$ dans le $CDCl_3$, BRUKER 400 MHz. HPLC colonne phase normale CHIRALCEL O-DH (colonne ODHOCE-CE026), éluant 9/1 Hexane/iPrOH, 20mn, longueur d'onde 190nm.

Temps de rétention 6,186 mn, pureté HPLC 91,7%.

**1H RMN (CDCl3, 400,13 MHz):** δ 0,62 (s, 3H, CH3), 0,78 (dd, 6H, 2CH3), 0,85 (d, 3H, CH3), 0,86-2,28 (m, 27H), 1,03 (s, 3H, CH3), 1,96 (s, 3H, CH3 acétyle), 3,47 (m, 1H, CH), 4,94 (td, 1H, CH), 5,13 (t, 1H, CH).

<u>Exemple 2: préparation du 7beta-tert-butyloxycarbonylcholestérol (composé 1.6)</u>

Le schéma réactionnel est représenté sur la Figure 2.

Le composé 1.6 est préparé à partir du composé 1.2 de l'exemple 1.

1) Préparation du composé 1.5

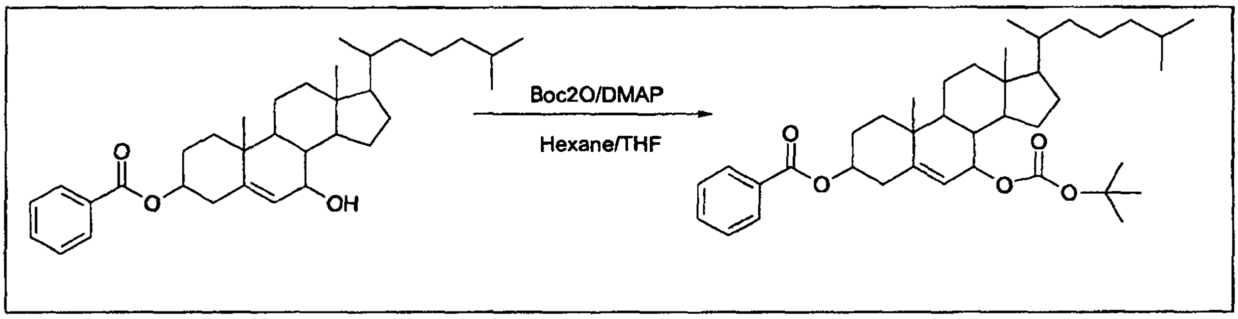


Les réactifs suivants ont été utilisés :

| | MW | Nb mol. | éq | Masse ou Volume |
|---|---|---|---|---|
| 7-β-hydroxycholestéryl benzoate | 505,76 | 1,29 mmol | - | 0,65 g |
| Boc2O | 218,25 | 2,8 mmol | 2,2 éq | 0,61 g |
| DMAP | 122,17 | 0,13 mmol | 0,1 éq | 0,016 g |
| Hexane/THF 5 :2 | | | | 42 ml |

Dans un ballon monocol de 100ml, on place le 7-β-hydroxycholesteryl benzoate, le solvant, la 2,6-diméthylaminopyridine puis l'anhydride de tert-butyloxycarbonyle. Ce mélange est agité à température jusqu'à complète solubilisation. Le suivi de l'avancement est réalisé par CCM dans un éluant 8/2 Hexane/AcOEt.

On ajoute 50 ml d'AcOEt et 10ml d'eau. La phase organique ainsi obtenue est séchée sur sulfate de sodium, filtrée puis évaporée sous pression réduite pour conduire à un résidu huileux.

Le résidu est repris dans l'AcOEt afin de préparer le dépôt solide. La purification est réalisée sur colonne de gel de silice dans un éluant Hexane/AcOEt 95/5.

Analyse : Analyse par RMN $^1H$ dans le $CDCl_3$.

**1H RMN (CDCl3, 400,13 MHz):** δ 0,59 (s, 3H, CH3), 0,77 (dd, 6H, 2CH3), 0,84 (d, 3H, CH3), 0,86-1,98 (m, 24H), 1,09 (s, 3H, CH3), 1,41 (s, 9H, 3CH3, t-Boc), 2,42 (m, 2H, CH2), 4,80 (m, 1H, CH), 4,91 (d, 1H, CH), 5,18 (s, 1H, CH), 7,34-7,97 (m, 5H, CHAr).

2) Préparation du composé 1.6

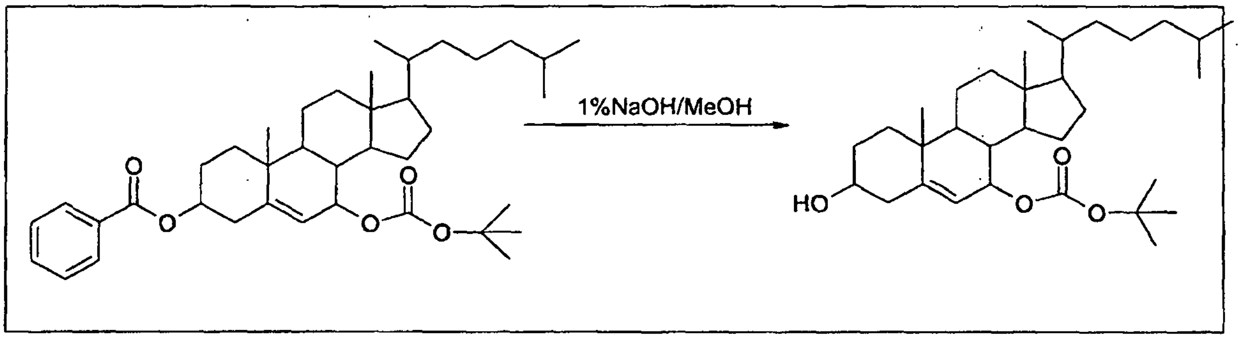


Les réactifs suivants ont été utilisés :

| | MW | Nb mol. | éq | Masse ou Volume |
|---|---|---|---|---|
| 7-β-t-butyloxycarbonyl-cholestéryl benzoate | 606,89 | 1,64 mmol | - | 1g |
| 1% NaOH MeOH | | | | 50 ml |

Dans un ballon monocol de 50ml, on place le 7-β-t-butyloxycarbonylcholestéryl benzoate et la solution 1% de soude dans le méthanol. Ce mélange est agité à température ambiante jusqu'à complète solubilisation. Le suivi de l'avancement est réalisé par CCM dans un éluant 7/3 Hexane/AcOEt. Afin de compléter la réaction le brut peut être chauffé à 40°C.

On ajoute 100 ml d'AcOEt et 20ml d'eau. La phase aqueuse est réextraite à l'AcOEt. Les phases organiques sont rassemblées, séchées sur sulfate de sodium, filtrées puis évaporées sous pression réduite pour conduire à un résidu huileux.

Le résidu est repris dans l'AcOEt afin de préparer le dépôt solide. La purification est réalisée sur colonne de gel de silice dans un éluant Hexane/AcOEt allant de 9/1 à 7/3. La colonne est lavée à l'AcOEt 100% afin de récupérer le 7-β-hydroxycholestérol formé.

**Analyse :** Analyse par RMN $^1H$ dans le $CDCl_3$.

**1H RMN (CDCl3, 400,13 MHz) :** δ 0,60 (s, 3H, CH3), 0,78 (dd, 6H, 2CH3), 0,84 (d, 3H, CH3), 0,91-2,29 (m, 27H), 0,97 (s, 3H, CH3), 1,41 (s, 9H, 3CH3, t-Boc); 3,47 (m, 1H, $CH_B$), 4,77 (td, 1H, $CH_C$), 5,17 (t, 1H, $CH_A$).

Exemple 3 : Préparation du 7-((*tert*-butoxycarbonyl)oxyl)-10,13-diméthyl-17-(6-méthylheptan-2-yl)-2,3,4,7,8,9,10,11,12,13,14,15,16,17-tétradécahydro-1*H*-cvclopentaralphenanthren-3-yl 2-(2-(((benzyloxy)carbonyl)amino)acétamido)-propanoate (molécule 1.a)

Nom simplifié: 3-benzyloxycarbonyl-glycinyl-alanyl-7-β-O-tert-butyloxycarbonyl-cholestérol

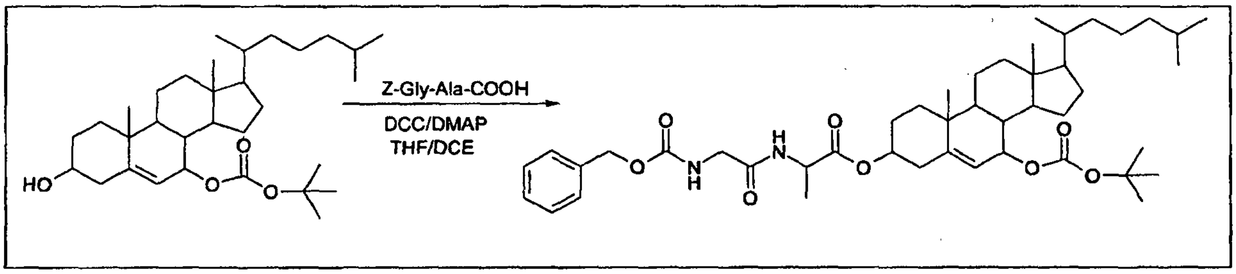


La molécule 1.a est préparée à partir du composé intermédiaire 1.6. Les réactifs suivants ont été utilisés :

| | MW | Nb mol. | éq | Masse ou Volume |
|---|---|---|---|---|
| 7-β-t-butyloxycarbonyl-cholestérol (composé 1.6) | 502 | 0,16 mmol | - | 80 mg |
| Z-Gly-Ala-COOH | 280,28 | 0,24 mmol | 1,5 éq | 68 mg |
| DCC | 206,3 | 0,24 mmol | 1,5 éq | 50 mg |
| DMAP | 122,17 | 0,24 mmol | 1,5 éq | 30 mg |
| THF/DCE 1:1 | | | | 6 ml |

Dans une bouteille Wheaton de 10ml, on place 80mg (0,16mmol) de 7-β-t-butyloxycarbonylcholestérol, 68mg (0,24mmol, 1,5éq) de dipeptide, 6ml de mélange de solvants (THF/DCE 1 :1), 50mg (0,24mmol, 1,5éq) de DCC, et 30mg (0,24mmol, 1,5éq) de DMAP. Le brut réactionnel est agité durant 24h à température ambiante. Le suivi de l'avancement est réalisé par CCM dans un éluant Hexane/Acétate d'éthyle 7/3.

On ajoute de 30 ml d'Acétate d'éthyle et 10 ml d'eau au brut réactionnel. La phase organique est séparée, séchée sur sulfate de sodium, filtrée puis évaporée sous pression réduite. Le résidu huileux obtenu est repris dans l'Acétate d'éthyle afin de préparer le dépôt solide.

La purification est effectuée sur colonne de gel de silice dans un éluant Hexane/Acétate d'éthyle 7/3, puis 6/4.

**Analyse :** Analyse par RMN du $^1H$ dans le $CDCl_3$.

**1H RMN (CDCl3, 400,13 MHz) :** δ 0,60 (s, 3H, CH3), 0,79 (dd, 6H, 2CH3), 0,83 (d, 3H, CH3), 0,94-1,88 (m, 24H), 0,98 (s, 3H, CH3), 1,19 (s, 3H, CH3 Ala), 1,40 (s, 9H, 3CH3, t-Boc), 2,27 (m, 2H, CH2), 3,83 (m, 2H, CH2), 4,47 (td, 1H, CH Ala), 4,47 (m, 1H, CH), 4,78 (td, 1H, CH), 5,07 (s, 2H, CH2 Gly), 5,22 (t, 1H, CH), 5,23 (m, 1H, NH), 6,41 (si, 1H, NH), 7,29 (m, 5H, CHAr).

Exemple 4 : Préparation du 7-acétoxy-10,13-diméthyl-17-(6-méthylheptan-2-yl)-2,3,4,7,8,9,10,11,12,13,14,15,16,17-tétradécahydro-1*H*-cyclopenta[*a*]phenanthren-3-yl 2-(2-(((penzyloxy)carbonyl)amino): acétamido)propanoate (molécule 1.b)

Nom simplifié : 3-benzyloxycarbonyl-glycinyl-alanyl-7-β-O-acétylcholestérol

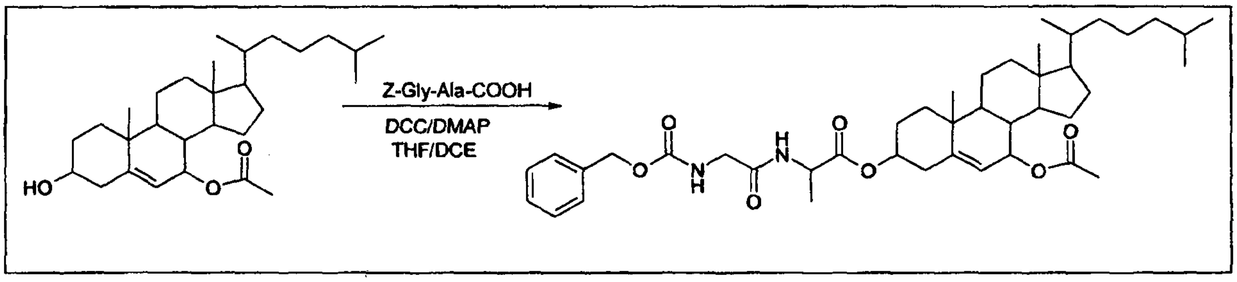


La molécule 1.b est préparée à partir du composé intermédiaire 1.4. Les réactifs suivants ont été utilisés :

| | MW | Nb mol. | éq | Masse ou Volume |
|---|---|---|---|---|
| 7-β-acétylcholestérol | 444 | 0,18 mmol | - | 80 mg |
| Z-Gly-Ala-COOH | 280,28 | 0,27 mmol | 1,5 éq | 76 mg |
| DCC | 206,3 | 0,27 mmol | 1,5 éq | 56 mg |
| DMAP | 122,17 | 0,27 mmol | 1,5 éq | 33mg |
| THF/DCE 1:1 | | | | 6 ml |

Dans une bouteille Wheaton de 10ml, on place 60mg (0,13mmol) de 7-β-acétylcholestérol, 70mg (0,19mmol, 1,5éq) de dipeptide, 6ml de mélange de solvants (THF/DCE 1 :1), 42mg (0,19mmol, 1,5éq) de DCC, et 25mg (0,19mmol, 1,5éq) de DMAP. Le brut réactionnel est agité durant 24h à température ambiante.

Le suivi de l'avancement est réalisé par CCM dans un éluant Hexane/Acétate d'éthyle 7/3.

On ajoute 30 ml d'Acétate d'éthyle et 10 ml d'eau au brut réactionnel. La phase organique est séparée, séchée sur sulfate de sodium, filtrée puis évaporée sous pression réduite. Le résidu huileux obtenu est repris dans l'Acétate d'éthyle afin de préparer le dépôt solide.

La purification est effectuée sur colonne de gel de silice dans un éluant Hexane/Acétate d'éthyle 7/3, puis 6/4.

**Analyse :** Analyse par RMN $^1H$ dans le $CDCl_3$.

**1H RMN (CDCl3, 400,13 MHz) :** δ 0,62 (s, 3H, CH3), 0,78 (dd, 6H, 2CH3), 0,84 (d, 3H, CH3), 0,91-1,83 (m, 25H), 1,01 (s, 3H, CH3), 1,17 (s, 3H, CH3 Ala), 1,94 (s, 3H, CH3 acétyle), 2,27 (m, 2H, CH2), 3,82 (m, 1H, CH), 4,47 (td, 1 H, CH Ala), 4,56 (m, 1 H, CH), 4,95 (td, 1H, CH), 5,06 (s, 2H, CH2 Gly), 5,17 (si, 1 H, CH), 5,37 (t, 1 H, CH), 6,49 (dl, 1H, NH), 7,24 (m, 5H, CHAr).

Exemple 5 : Préparation du 7-((*tert*-butoxycarbonyl)oxy)-10.13-diméthyl-17-(6-méthylheptan-2-yl)-2,3,4,7,8,9,10,11,12,13,14,15,16,17-tétradécahydro-1*H*-cyclopenta[*a*]phenanthren-3-yl 2,2-diméthyl-1,3-dioxolane-4-carboxylate (molécule 2.a)

Nom simplifié : 3-(S)-2,2-diméthyl-1,3-dioxolane-4-carboxyl-7-β-O-tert-butyloxycarbonyl- cholestérol

1) Préparation du groupement diméthyl-1,3-dioxolane-4-carboxylate

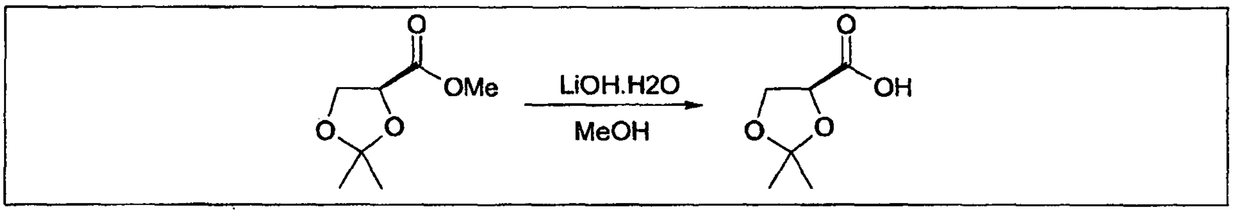


Les réactifs suivants ont été utilisés :

| | MW | Nb mol. | éq | Masse ou Volume |
|---|---|---|---|---|
| (-)-Méthyl (S)-2,2-diméthyl-1,3-dioxolane-4-carboxylate | 160,17 | 31,2 mmol | - | 5 g |
| LiOH.H2O | 41,96 | 78 mmol | 2,5 éq | 3,3 g |
| Méthanol | | | | 25 ml |

Dans un ballon de 50ml, on place l'acétal, le méthanol puis la lithine. Ce mélange est agité à température ambiante pendant 16h.

Le brut réactionnel est évaporé sous pression réduite. Le résidu obtenu est repris dans 75 ml d'eau. Cette phase est alors acidifiée à 0°C jusqu'à pH 1 à l'acide chlorhydrique 1N, puis extraite par 2 x 100ml d'acétate d'éthyle. Les phases organiques sont rassemblées, séchées sur sulfate de sodium, filtrées puis évaporées sous pression réduite pour conduire à un résidu huileux légèrement coloré.

Le résidu est directement introduit dans l'étape de couplage sans purification supplémentaire.

**Analyse :** Analyse par RMN $^1H$ dans le DMSO, BRUKER 400 MHz.

**1H RMN (CDCl3, 400,13 MHz) :** δ 1,32 (d, 3H, CH3), 1,42 (s, 3H, CH3), 4,14 (AB, 2H, CH2), 4,55 (dd, 1H, CH), 10,30 (sl, 1 H, OH), 7,24 (m, 5H, CHAr).

**Rendement :** 94%

2) Préparation de la molécule 2.a

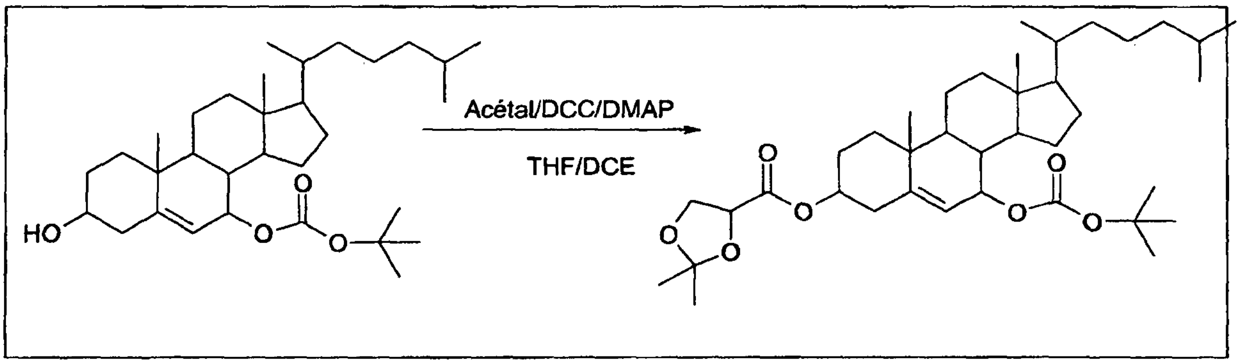


La molécule 2.a est préparée à partir du composé intermédiaire 1.4.

Les réactifs suivants ont été utilisés :

| | MW | Nb mol. | éq | Masse ou Volume |
|---|---|---|---|---|
| 7-β-acétylcholestérol | 444 | 3,4 mmol | - | 1,5 g |
| Acide 3-(S)-2,2-diméthyl-1,3-dioxolane-4-carboxylique | 146,17 | 10,1 mmol | 3 éq | 1,35 g |
| DCC | 206,3 | 10,1 mmol | 3 éq | 2,1 g |
| DMAP | 122,17 | 10,1 mmol | 3 éq | 1,24 mg |
| THF/DCE (1 :1) | | | | 50 ml |

Dans une bouteille Wheaton de 10ml, on place 50 mg (9,9mmol) de 7-β-t-butyloxycarbonylcholestérol, 6ml de mélange de solvants (THF/DCE 1 :1), et 16mg (11,9mmol, 1,2éq) d'acide 3-(S)-2,2-dimethyl-1,3-dioxolane-4-carboxylique.

On ajoute 24,5mg (11,9mmol, 1,2éq) de DCC et 14,5mg (11,9mmol, 1,2éq) de DMAP, avant d'agiter le brut réactionnel durant 24h à température ambiante. Le suivi de l'avancement est réalisé par CCM dans un éluant Hexane/Acétate d'éthyle 8/2. On chauffe 2h à 50°C afin de terminer la réaction.

On ajoute 30 ml d'acétate d'éthyle et 10 ml d'eau au brut réactionnel. La phase organique est séparée, séchée sur sulfate de sodium, filtrée puis évaporée sous pression réduite. Le résidu huileux obtenu est repris dans l'Acétate d'éthyle afin de préparer le dépôt solide.

La purification est effectuée sur colonne de gel de silice dans un éluant Hexane/Acétate d'éthyle 95/5, puis 9/1.

**Analyse :** Analyse par RMN $^1H$ dans le $CDCl_3$.

**1H RMN (CDCl3, 400,13 MHz):** δ 0,60 (s, 3H, CH3), 0,79 (dd, 6H, 2CH3), 0,83 (d, 3H, CH3), 0,74-1,97 (m, 25H), 0,98 (s, 3H, CH3), 1,33 (s, 3H, CH3 Acétal), 1,40 (s, 9H, 3CH3, t-Boc), 1,42 (s, 3H, CH3 Acétal), 2,29 (m, 2H, CH2), 4,08 (AB, 2H, CH2 Acétal), 4,48 (ddd, 1 H, CH Acétal), 4,63 (m, 1 H, CH), 4,78 (td, 1 H, CH), 5,22 (d, 1 H, CH).

<u>Exemple 6 : Préparation du 7-acétoxy-10,13-diméthyl-17-(6-méthylheptan-2-yl)-2,3,4,7,8,9,10,11,12,13,14,15,16,17-tétradécahydro-1H-cyclopenta[a]phenanthren-3-yl 2,2-diméthyl-1,3-dioxolane-4-carboxylate (molécule 2.b)</u>

Nom simplifié : 3-(*S*)-2,2-diméthyl-1,3-dioxolane-4-carboxyl-7-β-O-acétyl-cholestérol

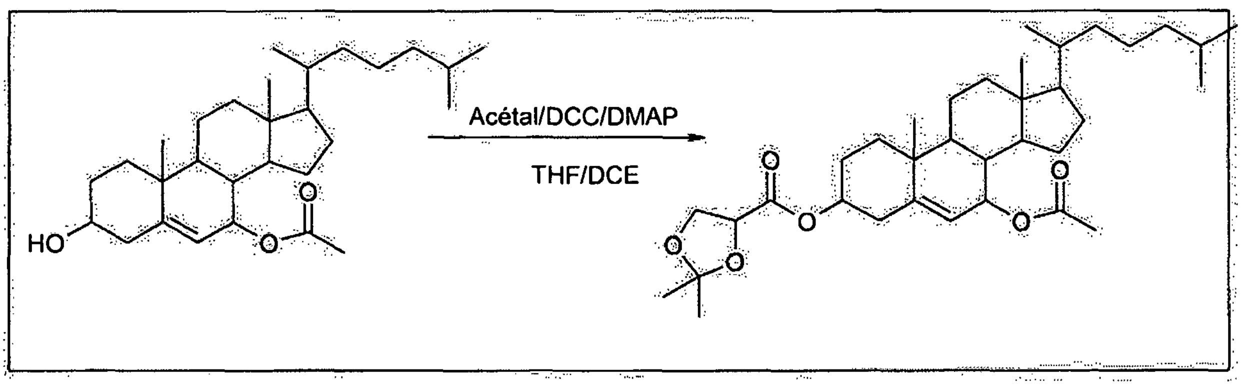


La molécule 2.b est préparée à partir du composé intermédiaire 1.4.

Les réactifs suivants ont été utilisés :

| | **MW** | **Nb mol.** | **éq** | **Masse ou Volume** |
|---|---|---|---|---|
| 7-β-acétylcholestérol | 444 | 3,4 mmol | - | 1,5 g |
| Acide 3-(*S*)-2,2-dimethyl-1,3-dioxolane-4-carboxylique | 146,17 | 10,1 mmol | 3 éq | 1,35 g |
| DCC | 206,3 | 10,1 mmol | 3 éq | 2,1 g |

(suite)

| | MW | Nb mol. | éq | Masse ou Volume |
|---|---|---|---|---|
| DMAP | 122,17 | 10,1 mmol | 3 éq | 1,24 mg |
| THF/DCE (1 :1) | | | | 50 ml |

Dans un ballon de 100ml, on place le cholestérol, le mélange de solvants et l'acide. On ajoute la DCC et la DMAP, avant d'agiter le brut réactionnel durant 24h à température ambiante. Le suivi de l'avancement est réalisé par CCM (TLC silica gel 60 F254, Merck) dans un éluant Hexane/AcOEt 8/2.

On ajoute 100 ml d'acétate d'éthyle et 50 ml d'eau au brut réactionnel. La phase organique est séparée, séchée sur sulfate de sodium, filtrée puis évaporée sous pression réduite.

Le résidu huileux obtenu est repris dans l'acétate d'éthyle afin de préparer le dépôt solide. Colonne de gel de silice (silice SDS 60A, 35-70μm) dans un éluant Hexane/AcOEt 95/5, puis 9/1.

**Rendement :** 68%

**Analyse :** Analyse par RMN $^{1}H$ et $^{13}C$ dans le $CDCl_3$ BRUKER 400 MHz. HPLC colonne phase normale CHIRALCEL O-DH (colonne ODHOCE-CE026), éluant 9/1 Hexane/iPrOH, 20mn, longueur d'onde 190nm.

Temps de rétention 5,291 mn, pureté HPLC 98,7%.

**1H RMN (CDCl3, 400,13 MHz) :** δ 0,62 (s, 3H, CH3), 0,79 (dd, 6H, 2CH3), 0,84 (d, 3H, CH3), 0,91-2,30 (m, 27H), 1,01 (s, 3H, CH3), 1,33 (s, 3H, CH3 Acétal), 1,42 (s, 3H, CH3 Acétal), 1,93 (s, 3H, CH3 acétyl), 4;09 (AB, 2H, CH2 Acétal), 4,48 (dd, 1 H, CH Acétal), 4,62 (m, 1 H, CH), 4,96 (td, 1 H, CH), 5,18 (d, 1 H, CH).

## II/ Activité biologique

### A/ Protocoles

Les protocoles suivants ont été utilisés pour toutes les expérimentations :

1) Cultures cellulaires

On a utilisé des plaques de culture de 96 puits (NUNC, USA) à fond plat en plastique traité culture cellulaire (Sigma-Aldrich, réf 114754) ; 1500 cellules sont ensemencées par puits dans 200 μl de milieu de culture. Au moment du traitement, 100 μl de milieu de culture pure, 100 μl de milieu de culture contenant de l'éthanol, ou des liposomes, ou 100 μl de milieu de culture contenant les principes actifs sont ajoutés par puits ; le volume final du milieu de culture, après traitement, est donc de 300 μl pour tous les puits.

Les cultures sont incubées dans un incubateur Sanyo (Japon, modèle, MCO-19AIC-UV) à 37°C et dans une atmosphère à 5% de CO2 et saturée en humidité. Les cellules sont observées au microscope inversé (Nikon elipse TS100, Japon) et les photos prises à l'aide d'une caméra (DIGITAL camera with c-mount ; LABOVER, France). La hotte de culture est une hotte PSM (thermo SCIENTIFIC, modèle HERA SAFE KS12, France).

Le temps du doublement cellulaire est calculé selon les formules :

$$\log2(a) = \log10(a) \times 3.32$$

$$\log2(b) = \log(b) \times 3.32$$

$$\text{Temps de doublement} = \log2(b) - \log2(a)$$

où (a) et (b) représentent le nombre de cellules aux temps a et b (b>a) (12).

Les passages sont effectués par dissociation à la trypsine (Type 3 de pancréas de bovin, Sigma, France). La dissociation des cellules se fait à température ambiante, pendant 30 minutes, avec une solution de Tyrode KCl 0,04 % (p/v) contenant 0,05% (p/v) de trypsine.

Après dissociation, les cellules sont suspendues dans le milieu de culture, approprié les cellules et comptées à l'aide d'une cellule de THOMA et diluées dans le même milieu pour obtenir 1500 cellules par puits.

*a) Lignées cellulaires*

On a utilisé les cellules de glioblastomes suivantes :

• Lignées C6.

La lignée cellulaire de type C6 a été obtenue par Benda et col. (13) à partir de tumeurs de cerveau de rat, induites par la N-méthylnitrosourée. Ce type cellulaire est utilisé comme modèle «in vitro» et « in vivo» pour évaluer le potentiel anti-GBM. Les lignées proviennent de l'ancien Centre de Neurochimie de Strasbourg (U44 INSERM et UPR 416 CNRS).

Le milieu de culture est composé de 70% de Minimum Essential Medium (MEM ; (Fischer Scientifique, réf 61100) et 30% de solution de Hanks (SIGMA, réf H 9269). Sont ajoutés au milieu de culture : du sérum de veau foetal (SVF; Ficher Scientifique, réf 10108165) à une concentration finale de 5% (V/V), une solution antibiotique de ciprofloxacine hydrochlorate 5μg/mL (EUROMEDEX, réf UC5074) et une solution de fungizone 2,5 μg/mL (INVITROGEN, réf 15290-026). Le temps de doublement de ce type cellulaire est de 17 h.

• Lignées GBM d'origine humaine.

Les lignées de glioblastome humain (GBM, lignée U-87 MG) et leur milieu de culture (Eagle's Minimum Essential Medium ou EMEM) proviennent de ATCC (USA, réf ATCC- HTB-14). Les cellules sont mises et maintenues en culture selon les recommandations de l'ATCC. Ces lignées sont couramment utilisées «in vitro» et «in vivo» pour tester le potentiel anti GBM. Le temps de doublement de ces lignées est de 16 h.

On a également utilisé des cultures primaires de cellules humaines suivantes :

• Cellules astrocytaires

Les cellules astrocytaires d'origine humaine utilisées proviennent de ScienCell, USA (ref 1800) ainsi que leur milieu de culture, composé de milieu basal contenant 2% (V/V) de sérum de SVF (ref 0010), des protéines de croissance astrocytaire (AGS, réf 1852) et une solution de pénicilline/streptomycine (réf 0503). Le temps de doublement de ces astrocytes humains est de 96 h.

• Cellules hépatiques

D'origine humaine, elles proviennent de ScienCell, USA (ref 50200) ainsi que le milieu de culture (ref 5201) qui contient 10% (v/v) de SVF. Le temps de doublement de ces cellules est de 24 h.

• Cellules rénales

D'origine humaine, ces cellules proviennent de ScienCell, USA (ref 4120) ainsi que le milieu de culture (ref 4101) qui contient 10% (v/v) de SVF. Le temps de doublement est de 96 h.

• Cellules cardiaques

D'origine humaine, ces cellules proviennent de ScienCell, USA (ref 6300) ainsi que le milieu de culture qui contient 10% (v/v) de SVF. Le temps de doublement est de 72 h.

• Cellules de muscle squelettique

D'origine humaine, elles proviennent de ScienCell, USA (ref 3500) ainsi que le milieu de culture (ref 3501) qui contient 10% (v/v) de SVF. Le temps de doublement pour ces cellules est de 72 h.

On a également utilisé des cultures de cellules cancéreuses d'origine humaine suivantes :

• Cellules cancéreuses du foie

Elles proviennent de ATCC (ref ATCC-HB-8065). Le milieu de culture est constitué de MEM (Gibco, USA ; ref 51200) et 10% (V /v) de SVF. Le temps de doublement est de 60 h.

• Cellules cancéreuses de prostate

Elles proviennent de ATCC (ref ATCC-HTB-81). Le milieu de culture est le même que celui utilisé pour les lignées cancéreuses de foie. Le temps de doublement est de 60 h.

• Cellules cancéreuses du sein

Elles proviennent de ATCC (ref ATCC-HTB-19). Le milieu de culture est le même que celui utilisé pour les lignées cancéreuses de foie. Le temps doublement est de 20 h.
Pour toutes ces cultures, les mises et les maintiens en culture ont été faits selon les recommandations des fournisseurs.

*b) Traitement des cultures*

Les principes actifs sont dissous soit dans de l'éthanol absolu (AnalaR, NORMAPUR, VWR, France) ou sous forme d'une solution liposomale, tel que 10 μl des solutions-mères diluées dans 990 μl et ensuite ajoutés dans les puits de culture (200 μl de milieu de culture) donnent des concentrations de 30,0 ; 15,0 ; 7,5 et 3,3 μM (volume final du milieu de culture par puits BI-GBM : 300 μl).
Lorsque les principes actifs sont en solution éthanolique, le milieu de culture contient 3,3% d'éthanol (v/v).

2) Préparation des liposomes

La méthodologie de base est décrite par Werthle et al. (10).
Brièvement, les composés à tester, à savoir les composés selon l'invention (principes actifs) ou le 7β-OHCH-C3-ester (un dérivé de 7beta-hydroxystérol estérifié en position 3 par un groupement oléate, dont la synthèse est décrite par Rakotoarivelo et al. (14) et utilisé à titre de contrôle), la phosphatidylcholine de soja (Sigma) ainsi que le cholestéryl-3-sulfate (Sigma) sont prélevés dans leurs solutions-stock faites de dichloroéthane pour les principes actifs et le 7β-OHCH-C3-ester, d'un mélange chloroforme : méthanol (9:1, v/v) pour la phosphatidylcholine et de chloroforme pour le cholestéryl-3-sulfate. Les rapports molaires sont 1 M/0.1 M/0.25 M pour la phosphatidylcholine, le cholestéryl-3-sulfate ainsi que le 7β-OHCH-C3-ester et les principes actifs respectivement.
Après évaporation, une solution de PBS saline, sans $Ca^{2+}$ et $Mg^{2+}$, pH7, 2 (BioRad) est ajoutée au composé sec. Le volume de tampon et la masse des produits sont ajustés pour que 20 ou 10 μl de liposomes ajoutés à 90 μl de milieu de culture donne les concentrations finales désirées. Les liposomes sont formés par la méthode d'extrusion avec le Liposofast (Sodexim, SA Muizon, France). La solution est passée 41 fois à travers des membranes de filtration (100 nm) en polycarbonate. Les liposomes sont stérilisés par filtration sur des membranes Millipore de 22 μm.

3) Mesure de l'activité et de la toxicité

On utilise les mêmes méthodes pour les deux cas. Pour l'activité, on utilise le potentiel anti-GBM des composés à tester et, pour la toxicité, celle-ci testée sur des cellules normales d'origine humaine maintenues « in vitro ».
Les méthodes de mesure suivantes sont utilisées :

*a) Comptage cellulaire*

On a utilisé une méthode de comptage cellulaire sur photo.
Par exemple, pour le cas des cultures en plaques de 96 puits et en fonction du grossissement du microscope utilisé (x100 ou x200), une photo prise représente un champ de vue avec un diamètre égal à 1/5 du diamètre d'un puits. Donc, le nombre total de cellules dans un puits est égal à 5 fois le nombre de cellules par photo. Cette technique a été comparée avec une technique standard (trypsinisation du tapis cellulaire, centrifugation des cellules, suspension des cellules dans une solution physiologique et comptage avec une cellule de THOMA) pour le cas des cellules C6. Les résultats obtenus sont identiques pour les deux méthodes.

*b) Dosage des protéines.*

Le milieu de culture est retiré de chaque puits et 50 μl de tampon de Laemmli (0,1 ml de Tris, 0,8 ml de glycérol, 1,6 mL de SDS 10%, 8 ml q.s.p eau ultra pure) sont ajoutés par puits.
Dans des puits contrôles, 10 μl d'une solution de gamme d'albumine de sérum de boeuf (BSA cristallisée, Sigma) sont ajoutés ; la gamme allant de 0 jusqu'à 20 μg par puits. Les puits sont ensuite complétés par 40 μl de tampon

de Laemmli. Enfin, 200 μl de la solution du réactif BCA sont ajoutés (Pierce, USA ; BCA Protein Assay Kit ; ThemoScientific, France).

Les plaques de culture sont incubées pendant 30 min dans une étuve à 37 °C. La densité optique pour chaque puits est lue et quantifiée par un lecteur de plaques (BioRad, USA, iMark Microplate reader 12222) à 570 nm.

*c) Viabilité cellulaire (test MTT)*

Ce test permet de détecter la respiration cellulaire, particulièrement celle de la mitochondrie. La solution-stock de sel de tétrazolium MTT (bromure de 3-(4,5-diméthylthiazol-2-yl)-2,5-diphenyl tétrazolium, Sigma -Aldrich, USA, ref M5655) est faite de 10mg de MTT/ml de tampon PBS (Phosphate Buffer Saline, SIGMA, ref D 1408). Cette solution est directement ajoutée au milieu de culture de chaque puits ; la concentration finale de MTT est de 25 μg/ml. La plaque est alors placée à l'étuve à 37°C pendant 1 h minimum.

Après l'apparition des grains bleus de formazan; produit principalement par le transport mitochondrial d'électrons, des photos ont été prises pour compter les cellules présentant une forte densité de grains de formazan ; cette technique est utilisée pour le cas des lignées U87-MG. Ensuite pour cette lignée et les autres cultures, les milieux sont éliminés et 100 μl de diméthylsulfoxide (DMSO, SDS CARLO ERBA, Italie) sont ajoutés pour dissoudre les dépôts de formazan.

La densité optique se lit et se quantifie par l'utilisation du lecteur de plaques (BioRad, iMark Microplate reader) à 490 nm.

La viabilité est parfois aussi testée par la méthode d'exclusion du trypan bleu.

4) Immunomarquage des cellules et prises de vue

Les anticorps primaires anti-CD133 (poly, Abnova), anti-GFAP (poly, Sigma), anti-NFL (mono, Santa Cruz) et anti-fibroblast (ERTR7, Santa Cruz) sont dilués à 1/100 pour les trois premiers anticorps et à 1/50 pour le dernier.

Les anticorps secondaires correspondant à la reconnaissance des anticorps primaires sont des anticorps anti-lapin couplés à la peroxidase (chèvre, Sigma), anti-souris couplés au Dylight 488 (mouton, Sigma), anti-souris couplés au Dylight 488 (mouton, Sigma) et anti-rat couplés au FITC (lapin, Sigma). Les dilutions sont 1/4000, 1/1000, 1/4000 et 1/400 respectivement.

Les cellules sont perméabilisées pendant 5 min avec 0,1% de Triton X-100 (Sigma) dans du PBS (Fisher Scientific). Les sites non spécifiques sont bloqués avec 2% de BSA (Sigma) dans du PBS. Les incubations sont réalisées pendant 1 h à température ambiante, 48 h à 4°C, 24 h à 4 °C et 1 h à température ambiante pour les anticorps anti-CD133, anti-GFAP, anti-NFL et anti-Fibroblast respectivement. L'incubation avec les anticorps secondaires se fait durant 1hr à température ambiante. Les cellules CD133 positives sont révélées par le système DAB/H2O2 (Sigma), et observées au microscope photonique. La fluorescence ainsi que les prises de vue sont réalisées à l'aide d'un microscope à épifluorescence (Axiovert, Zeiss, Allemagne).

5) Extraction des lipides et identification des oxystérols

Les cellules sont lavées avec NaCl 9 °/°°, récoltées mécaniquement, suspendues dans un tampon Tris-HCl (10 mM ; pH 7,4) et homogénéisées avec un Potter (1000 tours / minute et 13 va-et-vient). L'homogénéisation se fait dans de la glace. L'extraction se fait à 4°C après avoir ajouté 19 volumes de chloroforme: méthanol (2 :1,v/v) pour 1 volume de suspension cellulaire selon Folch et al. (13). La phase organique contenant de l'eau est « cassée » avec 0,2 volume de de KCl à 0,74% (p/v).

Après évaporation de la phase organique au rotovapor (Buchi, R-215, Suisse) et le résidu lipidique repris dans du chloroforme. Les couches CCM sont prélavées avec du chloroforme : méthanol (1 :1, v/v) et activées à 100°C durant 1 h. Les lipides sont élués avec le système Ether de Pétrole (point d'ébullition : 60-70°C) : Ether: Acide Acétique (80 :20 :1,3, v/v/v). Les standards déposés sont du cholestérol, du 7-céto-cholestérol, du 7beta-OHCH, du 7beta-OHCH-C3-ester et de la molécule 2.b. Les lipides et standards sont révélés par le réactif de Maccala. Les Rf sont de 0,21, 0,09, 0,08, 0,27 et 0,23 pour le cholestérol, le 7-céto-chlestérol, le 7beta-OHCH, le 7beta-OHCH-C3-ester et la molécule 2.b respectivement. Des gammes de calibration allant de 0,5 à 2μg pour chaque standard sont effectuées séparément. L'intensité des spots d'intérêt sont calculés par rapport aux standards après «scan» des couches minces de silice révélées.

Les solvants utilisés sont de grade AnalAR ou HPLC.

**B/ Activité anti-GBM « in vitro »**

B.1. Le modèle animal

On a utilisé des cultures de cellules C6 (rat) dans les conditions opératoires mentionnées plus haut (partie II, A).

Exemple 7 : Activité anti-GBM « in vitro » sur des cultures de cellules C6 (rat)

Les résultats obtenus sont résumés dans le tableau 1 ci-dessous ainsi que sur la figure 3.

Tableau 1

| Molécules testées (éthanol 3%) | 1.a (ex. 3) 15 μm | 2.a (ex. 5) 15 μm | 1.a puis 2.a 15μm + 7,5 μm |
|---|---|---|---|
| Cellules résiduelles vivantes | 3% | 100% | 0% |

La Figure 3 (grossissement x 200) montre l'aspect des cultures de cellules C6 dans les conditions opératoires mentionnées plus haut (partie II, A) en présence de la molécule 1.a (ex.3) ou après traitement séquentiel avec la molécule 1.a puis la molécule 2.a (ex.5), à 19 jours et à 35 jours.

Les résultats (tableau 1) montrent que la molécule 1.a, sous forme éthanolique, est cytotoxique et ceci après 19 jours de traitement. A 15 μM de 1.a et après 14 jours de traitement, le nombre de cellules, le taux de protéines et le test MTT diminuent de 30% pour atteindre une baisse de 97%, au moins, à 19 jours. A 19 jours de traitement on observe aussi une dose dépendance pour 7,5 et 3,3 μM de 1.a et à 15μM on observe une augmentation du rapport MTT/cellule de 20% par rapport aux cultures contrôles.

Afin d'éliminer les cellules résiduelles, un traitement séquentiel a été utilisé. Les cellules sont d'abord traitées avec 15 μM de molécule 1.a et au 19$^{ème}$ jour de traitement, la molécule 2.a est ajoutée à une concentration de 7,5 μM.

Aucune cellule viable n'est observée ; seules des «cadavres» cellulaires adhèrent au substrat solide de la culture cellulaire (fond du puits) comme le montre la Figure 3.

En résumé, un traitement séquentiel par la molécule 1.a, d'abord, et, ensuite, ensuite par la molécule 2.a, montre une efficacité totale envers ce type cellulaire. Les observations indiquent une augmentation de la respiration globale des cellules avant leur destruction.

B.2. Le modèle humain : activité anti-GBM « in vitro » sur des cultures de cellules humaines (lignée U-87 GBM)

B.2.1 Définition du modèle « in vitro » et de l'expression des résultats

a) Caractérisation des différents types cellulaires

Les cultures de cellules U-87GM ont été réalisées dans les conditions opératoires décrites plus haut (partie II, A).

Les cultures sont composées de deux composantes cellulaires : un tapis cellulaire composé de cellules se comportant comme des cellules normales (non cancéreuses) et des amas cellulaire composés des cellules de type GBM

La Figure 4 montre une image photonique (X100) caractéristique de la culture. On distingue un tapis cellulaire (TC) sur lequel viennent se fixer des amas cellulaires (AC) en forme de demi-sphère. La Figure 5 (X200) montre, par immuno-marquage, que les cellules CD133+ (cellules souches), décrites dans les GBM humains sont localisées dans les amas cellulaires et non dans le TC. Des marquages à l'immunofluorescence montrent la présence de GFAP, marqueur d'astrocytes normaux (Figures 6 et 7; x 200) (16) dans TC et AC (Figures 6 et 7; x 200) et de neurofilaments dans le TC et AC. En revanche, le marquage spécifique de fibroblastes, cellules à fort potentiel de multiplication, n'est retrouvé que pour AC. L'observation photonique montre clairement que les cellules des AC se divisent très rapidement (temps de doublement de 96 h, au moins), alors que les cellules composant les AC ont un temps de doublement de 16 h.

Ces observations justifient la méthode du comptage cellulaire (cellules marquées ou non par les grains de formazan) qui a été développée ; en effet, le comptage après trypsinisation ne permet pas de distinguer entre les cellules de TC et de AC. Les images montrent aussi que seuls les AC ont un caractère GBM (temps de doublement court, présence de cellules souches et de fibroblastes). Les résultats obtenus sont donc ceux de l'effet des molécules testées sur AC.

b) Expression des résultats

Les résultats sont exprimés selon les paramètres suivants :

(i) Efficacité des molécules selon l'invention

- Quantification des cellules résiduelles, les cellules souches en particulier.
- Efficacité des molécules selon l'invention dans le temps.

Le milieu de culture contenant les molécules selon l'invention est remplacé par du milieu frais et la multiplication cellulaire « de novo » est examinée : aucune multiplication « de novo » signifie une destruction totale des cellules GBM, y compris les cellules souches.

(ii) Effet des molécules sur la respiration globale des cellules GBM.
(iii) Comparaison des résultats obtenus avec les molécules selon l'invention avec ceux obtenus avec 7β-OHCH-C3-ester (contrôle).

B.2.2 Résultats

On a utilisé des cultures de cellules U87-MG (humain) dans les conditions opératoires mentionnées plus-haut (partie II, A).
Le 7beta-OHCH-C3-ester a été utilisé comme contrôle.

Exemple 8 : étude de l'activité anti-GBM « in vitro » de la molécule 2.a (ex.5) sous forme liposomale

Les résultats obtenus sont rapportés dans le tableau 2 ci-dessous.

Tableau 2

| | Molécule 2.a (15 μM) sous forme liposomale | | 7β-OHCH-C3-ester (80 μM) sous forme liposomale | |
|---|---|---|---|---|
| Jours de traitement | Protéines | MTT/cellule | Protéines | MTT/cellule |
| 6 | 70 | 100 | 70 | 100 |
| 8 | 25 | 140 | 20 | 100 |
| 13 | 15 | 120 | 15 | 100 |
| 15 | 0 | 0 | 15 | 100 |

Ces résultats sont la moyenne de trois expériences faites en triplicat. Les résultats sont exprimés en % par rapport aux contrôles.
Les observations montrent que 15 μM de la molécule 2.a, sous forme liposomale, réduit à néant la présence des cellules GBM (AC) au bout de 15 jours de traitement.
Seules les cellules à division lente (TC) restent. L'effet n'est pas dose-dépendant. Le 7β-OHCH-C3-ester n'agit pas sous forme éthanolique sur les GBM (AC) (voir aussi l'exemple 9), et n'agit que sous forme liposomale, et ceci à 80 μM. Son efficacité n'est pas meilleure, voire diminue, en augmentant la dose de 7β-OHCH-C3-ester sous forme liposomale.
Cependant, si au 13$^{ème}$ jour de traitement, le milieu de culture contenant le 7β-OHCH-C3-ester est enlevé et est renouvelé par un milieu de culture frais ne contenant pas cette drogue, on observe une multiplication cellulaire et, en parallèle, une augmentation du test MTT au bout de deux jours ; cette augmentation est de 40%. Ceci n'est pas le cas pour la molécule 2.a :en l'absence de cette molécule, aucune multiplication cellulaire n'est observée.
Le tableau 2 montre également une forte augmentation de MTT/cellule (140% par rapport aux contrôles) au moment où les cellules disparaissent en masse (8 jours de traitement). Ceci n'est pas le cas pour le 7β-OHCH-C3-ester liposomale : même à 80μM, le rapport MTT/cellule ne varie pas.
Cette observation montre une action différente entre les deux molécules : la respiration globale cellulaire augmente avant la mort massive des cellules. Ceci n'est pas le cas pour le 7 β-OHCH-C3-ester.

Exemple 9 : étude de l'activité anti-GBM « in vitro » de la molécule 2.b (ex.6) sous forme éthanolique

Les résultats obtenus sont rapportés dans le tableau 3 ci-dessous.

Tableau 3

| | Molécule 2.b (30 μM) sous forme éthanolique | | 7β-OHCH-C3-ester (30 μM) sous forme éthanolique | |
|---|---|---|---|---|
| Jours de traitement | Protéines | MTT/cellule | Protéines | MTT/cellule |
| 4 | 100 | 100 | 100 | 100 |
| 6 | 100 | 100 | 100 | 100 |
| 9 | 90 | 160 | 100 | 100 |
| 15 | 50 | 130 | 100 | 100 |
| 22 | 0 | 0 | 100 | 100 |

Ces résultats sont la moyenne de trois expériences faites chacune en triplicat. Les résultats sont exprimés en % par rapport aux contrôles. Au-dessus de 30μM, le 7β-OHCH-C3-ester n'est plus soluble dans l'éthanol.

Les observations montrent que la molécule 2.b est totalement efficace à 30 μM sous forme éthanolique : il ne reste aucune cellule GBM, même après élimination du principe actif. Comme pour la molécule 2.a, sous forme liposomale, la respiration cellulaire augmente avant la mort cellulaire. Ceci n'est pas le cas pour le 7β-OHCH-C3-ester éthanolique, aucune activité anti-tumorale n'est observée.

De plus, un renouvellement de milieu de culture avec un milieu frais ne contenant pas le composé 2.b à 22 jours n'entraine aucune multiplication cellulaire.

Exemple 10 : immunomarquage des cellules souches CD133+ par le système A/B peroxydasse

L'immunomarquage est réalisé comme décrit plus haut (partie II, A).

Les résultats obtenus sont rapportés dans le tableau 4 ci-dessous.

Tableau 4

| Jours de traitement | Cellules CD133+ | | |
|---|---|---|---|
| | Cultures non traitées (Contrôles) | Molécule 2.b (30 μM) sous forme éthanolique | 7β-OHCH-C3-ester (80 μM) sous forme liposomale |
| 4 | 100 | 100 | 100 |
| 22 | 100 | 0 | 50 |

Ces résultats sont la moyenne de deux expériences faites chacune en triplicat. Les résultats sont exprimés en % de cellules CD133 positives par rapport aux contrôles. Ces expériences sont indépendantes de celles décrites dans les exemples 8 et 9.

Comme pour les observations décrites dans les tableaux 2 et 3, le 7β-OHCH-C3-ester liposomale et la molécule 2.b éthanolique diminuent de 85% et de 100% le taux de protéines par rapport aux cellules contrôles non traitées.

Les résultats montrent que les cellules souches sont totalement détruites par la molécule 2.b Ceci n'est pas le cas pour le 7β-OHCH-C3-ester, même administré sous forme liposomale.

La figure 9 (immuno-marquage des cellules $CD133^+$) montre clairement leur disparition après 22 jours de traitement. Ceci n'est pas le cas pour le 7β-OHCH-C3-ester sous forme liposomale (Figure 10, immuno-marquage des cellules $CD133^+$) ; dans ce cas, 50% des cellules $CD133^+$ subsistent parmi les cellules résiduelles. Un renouvellement de milieu de culture avec un milieu frais ne contenant pas le composé 2.b à 22 jours n'entraine aucune apparition de cellules souches CD133+.

Exemple 11 : étude du devenir de la molécule 2.b (ex. 6) sous forme éthanolique « in vitro » dans les GBM d'origine humaine

L'extraction et l'analyse des lipides à partir de GBM traités par 30 μM de la molécule 2.b, sous, forme éthanolique, ne montre aucune présence de 7beta-OHCH-C3-ester après 24 h ou 10 jours de traitement, ce dernier temps étant celui où la mort cellulaire est déclenchée. On observe, cependant, 0 ,12% et 0,18% de la molécule 2.b transformée en 7beta-OHCH après 1 jour et 10 jours de traitement respectivement. Les expériences contrôles montrent que ces très

faibles taux de 7beta-OHCH n'induisent aucune mort des GBM.

Exemple 12 : étude de la toxicité

La toxicité a été testée « in vitro » sur différents types cellulaires normaux d'origine humaine.

*a) Sur l'astrocyte*

Les cellules utilisées sont des cellules d'origine humaine (ScienCell, USA, ref 1800), mentionnées plus haut (partie II, A).

Le type astrocytaire est validé par la présence de GFAP, marqueur type des astrocytes normaux (Fig 11 ; x 200).

Les molécules 2.a (forme liposomale) et 2.b (forme éthanolique) ne sont pas toxiques sur les cultures primaires astrocytes humains normaux (non cancéreux) à 30 μM et, ceci après 30 jours de traitement.

*b) Sur d'autres cellules*

Les cellules utilisées sont des cellules de foie (ScienCell, USA, ref 50200), de rein (ScienCell, USA, ref 4120), de muscle squelettique (ScienCell, USA, ref 3500) et de cellules cardiaques (ScienCell, USA, ref 6300), d'origine humaine, mentionnées plus haut (partie II, A).

Les molécules 2.a (forme liposomale) et 2.b (fome éthanolique) ne sont pas toxiques à 30 μM et au bout de 30 jours de traitement, au moins, sur des cultures primaires de cellules de foie, de rein, de muscle squelettique et de cellules cardiaques d'origine humaine.

Exemple 13 : étude de l'activité de la molécule 2.b (ex.6) «in vitro» sur d'autres cancers

Les cellules cancéreuses utilisées sont des cellules cancéreuses de foie, (ref ATCC-HB-8065) de prostate (ref ATCC-HTB-81) ou de sein (ref ATCC-HTB-19), d'origine humaine, mentionnées plus haut.

A 30 μM et sous forme éthanolique, la molécule 2.b ne montre aucun effet sur la division et la respiration cellulaire de cellules cancéreuses de foie, de prostate ou de sein d'origine humaine.

Bibliographie

1. Kando T, Setoguchi T, Taga T.PNAS. 2004, 101 : 781-786.
2. Zhou Y, Zhou Y, Shingu T, Feng L, Chen Z, Ogasawara M, Keating MJ.J. Biol. Chem. 2011, 286 : 32843-32853).
3. Warburg O.Biochemische Zeitscrift. 1923, 142: 317-333.
4. Tennant DA, Duran RV, Gottlieb E.Nature Reviews Cancer. 2010, 10: 267-277.
5. Cheng KP, Nagano H, Bang L, Ourisson G, Beck JP.Journal of Chemistry Research (M). 1977, 217:2501-2521.
6. Carvalho JFS, Cruz Silva MM, Moreira JN, Simoes S, Sa e Melo ML.Journal of Médicinal Chemistry. 2011, 54: 6375-6393.
7. Kupferberg A, Teller G, Behr P, Leray C, Urban PF, Vincendon G, Mersel M. Biochim Biophys Acta. 1989, 1013: 231-23.
8. Kupferberg A, Behr P, Mersel M.Biochim Biophys Acta. 1990. 1046: 106-109.
9. Adamczyck M, Scherrer E, Kupferberg A, Malviya AN, Mersel M.Journal of Neuroscience Research.1988,53:38-50.
10. Werthle M, Bochelen D, Adamczyck M, Kupferberg A, Poulet P, Chambron J, Lutz P, PrivatA, Mersel M.Cancer Research. 1994, 54: 998-1003.
11. Clarion L, Schindler M, de Weille J, Lolmède K, Laroche-Clary A, Uro-Coste E, Robert J, Mersel M, Bakalara N.Biochemical Pharmacology. 2012, 83: 37-46.
12. Manford K, Patterson JR.Methods in Enzymology. 1979, 58: 150.
13. Benda P, Lightbody J, Sato G, Levine L, Sweet W.Science.1968, 161: 370-371.
14. Rakotoarivelo C, Adamczyck M, Desgeorges M, Langley K, Lorentz JG, Mann A, Ricard D, Scherrer E, Privat A, Mersel M.Anticancer Research. 2006, 26:2053-2062.
15. Folch J, Lees M, Sloane-Stanley GHJournal of Biological Chemistry.1957, 226: 497-509.
16. Sensenbrenner M, Devilliers G, Bock E, Porte A.Differentiation.1980, 17: 51-61.

## Revendications

1. Composé de formule (I)

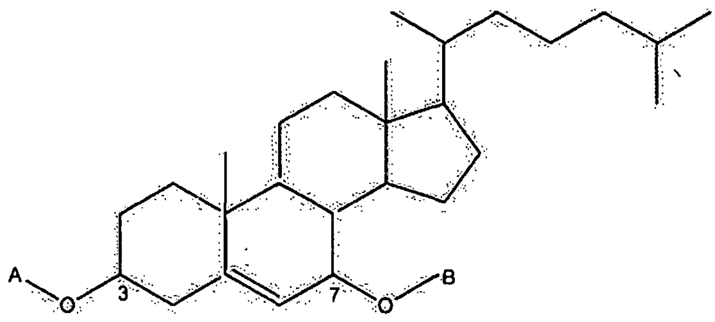

(I)

dans laquelle

- A représente
un groupement $-(R_1)_n-$ dans lequel $R_1$ est un reste d'acide aminé lié par son extrémité C-terminale et n= 1 à 3, chacun des $R_1$ étant identique ou différent, dans lequel l'extrémité N-terminale dudit acide aminé est non substituée ou substituée par un groupe $-C-(O)-R_2$ dans lequel $R_2$ est un groupe arylalkyl mono-ou pluri-cyclique en $C_6$-$C_{14}$ ; un groupe hétéroarylalkyl mono-ou pluri-cyclique en $C_5$-$C_{14}$ pouvant comporter un ou plusieurs hérétoatomes, identiques ou différents; un groupe arylalkyloxy mono-ou pluri-cyclique en $C_6$-$C_{14}$ ou un groupe hétéroarylalkyloxy mono- ou pluri-cyclique en $C_5$-$C_{14}$ pouvant comporter un ou plusieurs hérétoatomes, identiques ou différents,
un groupement $-C(O)-NH-R_3$ ou $-C(S)-NH-R_3$ dans lequel $R_3$ est l'hydrogène ; un groupe alkyle en $C_1$-$C_{12}$, linéaire ou ramifié, non substitué ou substitué par un groupement choisi parmi OR, NRR', NHR et SR, où est R et R', indépendamment, représentent l'hydrogène, un alkyle linéaire en $C_1$-$C_{12}$ ou un aryle non substitué; un groupe aryle, non substitué ou substitué par un groupement choisi parmi OR, NRR', NHR et SR, tels que définis ci-dessus; un groupe acyle ; un groupe formyle ; un groupe sulfonyle ; un groupe sulfinyle ; un groupe silyle non substitué ou substitué non substitué ou substitué par un groupe aryle mono- ou pluri-cyclique en $C_6$-$C_{14}$, non substitué ou substitué par au moins un alkyle en $C_1$-$C_6$ linéaire ou ramifié; un groupe allyle ; un reste de sucre ; un groupe-$C(O)-OR_4$ dans lequel $R_4$ est un alkyle en $C_1$-$C_{12}$, linéaire ou ramifié, non substitué ou substitué par un groupement choisi parmi OR, NRR', NHR et SR, tels que définis ci-dessus; un groupe amide ; un groupe thioamide ; un groupe sulfonamide,
un groupement $-C(O)-R_5$ dans lequel $R_5$ est un hétérocycle saturé en $C_5$-$C_{14}$ comprenant 1 ou 2 hétéroatomes, non substitué ou substitué par au moins un alkyle en $C_1$-$C_6$ linéaire ou ramifié ou un groupement choisi parmi OR, NRR', NHR et SR, où est R et R', indépendamment, représentent l'hydrogène, un alkyle en $C_1$-$C_{12}$ ou un aryle non substitué.
- B représente un groupement $-C(O)-R_6$, dans lequel $R_6$ est l'hydrogène ; un alkyle en $C_1$-$C_{12}$, de préférence en $C_1$-$C_{6,}$ linéaire ou ramifié, non substitué ou substitué par un groupement choisi parmi OR, NRR', NHR et SR, tels que définis ci-dessus; un groupe aryle, non substitué ou substitué par un groupement choisi parmi OR, NRR', NHR et SR, tels que définis ci-dessus ; ou encore $R_6$, représente $OR_7$, dans lequel $R_7$, est un alkyle en $C_1$-$C_{12,}$ de préférence en $C_1$-$C_{6,}$ linéaire ou ramifié.

2. Composé de formule (I) selon la revendication 1, dans lequel au moins une des conditions suivantes est remplie :

- A représente un groupement $-(R_1)_n-$ dans lequel $R_1$ est un reste d'acide aminé et n = 2 ;
- A représente un groupement $-(R_1)_n-$ dans lequel $R_1$ est un reste d'acide aminé, n= 2 et l'extrémité N-terminale dudit acide aminé est substituée par un groupe arylalcoxycarbonyl, en particulier benzyloxycarbonyl ;
- A représente un radical alanyl lié à un radical glycinyl ; éventuellement substitué sur son extrémité N-terminale par un groupe arylalcoxycarbonyl, en particulier benzyloxycarbonyl;
- A représente un radical méthionyl lié à un radical glycinyl, éventuellement substitué sur son extrémité N-terminale par un groupe arylalcoxycarbonyl, en particulier benzyloxycarbonyl ;

3. Composé de formule (I) selon la revendication 1, dans lequel A représente un groupement $-C(O)-R_5$ dans lequel $R_5$ est un groupe 2,2 diméthyl-1,3-dioxolane.

**4.** Composé de formule (I) selon l'une des revendications 1 à 3, dans lequel B représente un groupement acyl dans lequel le groupe alkyl est en $C_1$-$C_6$, en particulier acétyl, ou encore un groupement alcoxycarbonyl dans lequel le groupe alkyl est en $C_1$-$C_6$, en particulier un groupement *tert*-butoxycarbonyl.

**5.** Composé de formule (I) selon la revendication 1, **caractérisé en ce qu'**il est choisi parmi les composés suivants :

- le 7-((*tert*-butoxycarbonyl)oxyl)-10,13-diméthyl-17-(6-méthylheptan-2-yl)-2,3,4,7,8,9,10, 11,12,13,14, 15,16, 17-tétradécahydro-1*H*-cyclopenta[a]phenanthren-3-yl 2-(2-(((benzyloxy)carbonyl)amino)-acétamido)propanoate (molécule 1.a) ;
- le 7-acétoxy-10,13-diméthyl-17-(6-méthylheptan-2-yl)-2,3,4,7,8,9,10,11,12,13,14,15,16,17-tétradécahydro-1 H-cyclopenta[a]phenanthren-3-yl 2-(2-(((benzyloxy)carbonyl)amino)-acétamido)propanoate (molécule 1.b) ;
- le 7-((tert-butoxycarbonyl)oxy)-10,13-diméthyl-17-(6-méthylheptan-2-yl)-2,3,4,7,8,9,10,11,12,13,14,15,16, 17-tétradécahydro-1H-cyclopenta[a]phenanthren-3-yl 2,2-diméthyl-1,3-dioxolane-4-carboxylate (molécule 2.a) ;
- le 7-acétoxy-10,13-diméthyl-17-(6-méthylheptan-2-yl)-2,3,4,7,8,9,10,11,12,13,14,15,16,17-tétradécahydro-1H-cyclopenta[a]-phenanthren-3-yl 2,2-diméthyl-1,3-dioxolane-4-carboxylate (molécule 2.b).

**6.** Procédé de préparation d'un composé de formule (I) selon l'une quelconque des revendications 1 à 5, **caractérisé en ce qu'**il comprend les étapes suivante :

- protection de la fonction hydroxyle en position 3 du cholestérol par un groupement protecteur,
- introduction d'une fonction cétone en position 7,
- réduction de la fonction cétone en fonction hydroxyle,
- introduction d'un groupement protecteur sur la fonction hydroxyle en position 7, correspondant au groupement B, et
- déprotection de la fonction hydroxyle en position 3.

**7.** Procédé selon la revendication 6, **caractérisé en ce que**, après déprotection, la fonction hydroxyle en position 3 peut être substituée par le groupement A souhaité.

**8.** Composition pharmaceutique comprenant au moins un composé de formule (I) selon l'une quelconque des revendications 1 à 5 et un véhicule pharmaceutiquement acceptable.

**9.** Composition pharmaceutique selon la revendication 8, **caractérisée en ce que** ledit composé de formule (I) est utilisé comme seul principe actif, ou en association avec un agent anti-cancéreux.

**10.** Composition pharmaceutique selon l'une des revendications 8 ou 9, **caractérisée en ce qu'**elle consiste en un liposome ou en une solution alcoolique, d'au moins un composé de formule (I), seul ou en association avec un autre principe actif.

**11.** Composé de formule (I) selon l'une quelconque des revendications 1 à 5, pour son utilisation dans le traitement de maladies impliquant des cellules astrocytaires transformées.

**12.** Composé de formule (I) selon l'une quelconque des revendications 1 à 5, pour son utilisation dans le traitement du glioblastome multiple.

**13.** Composé de formule (I) selon l'une quelconque des revendications 1 à 5, pour son utilisation dans le traitement de maladies impliquant des cellules astrocytaires transformées, ledit traitement étant un traitement séquentiel comprenant au moins une étape d'administration d'un premier composé de formule (I) et au moins une étape d'administration d'un deuxième composé de formule (I), différent du premier.

**14.** Composé de formule (I) selon l'une quelconque des revendications 1 à 5, pour son utilisation dans le traitement du glioblastome multiple, ledit traitement étant un traitement séquentiel comprenant au moins une étape d'administration d'un premier composé de formule (I) et au moins une étape d'administration d'un deuxième composé de formule (I), différent du premier.

**15.** Composition pharmaceutique selon l'une des revendications 8 à 10, **caractérisée en ce que** ledit composé de formule (I) est le 7-acétoxy-10,13-diméthyl-17-(6-méthylheptan-2-yl)-2,3,4,7,8,9,10,11,12,13,14,15,16,17-tétradécahydro-1H-cyclopenta[a]-phenanthren-3-yl 2,2-diméthyl-1,3-dioxolane-4-carboxylate (molécule 2.b).

**16.** Composé de formule (I) selon la revendication 5 , pour son utilisation selon la revendication 14, **caractérisée en ce que** le premier composé de formule (I) est le 7-((*tert*-butoxycarbonyl)oxyl)-10,13-diméthyl-17-(6-méthylheptan-2-yl)-2,3,4,7,8,9,10,11,12,13,14,15,16,17-tétradécahydro-1*H*cyclopenta[a]phenanthren-3-yl 2-(2-(((benzyloxy)carbonyl)amino)-acétamido)propanoate (molécule 1.a) et le deuxième composé de formule (I) est le 7-((tert-butoxycarbonyl)oxy)-10,13-diméthyl-17-(6-méthylheptan-2-yl)-2,3,4,7,8,9,10,11,12,13,14,15,16,17-tétradécahydro-1H-cyclopenta[a]phenanthren-3-yl 2,2-diméthyl-1,3-dioxolane-4-carboxylate (molécule 2.a).

## Patentansprüche

**1.** Verbindung der Formel (I)

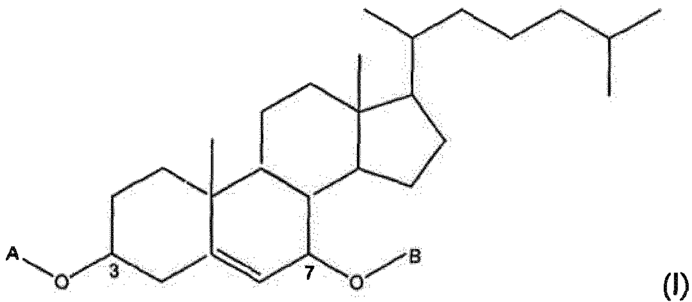

(I)

worin

- A steht für
eine $-(R_1)_n$-Gruppierung, worin $R_1$ ein Aminosäurerest ist, der über sein C-terminales Ende angebunden ist, und n = 1 bis 3 ist, worin jeder $R_1$ identisch oder unterschiedlich ist, worin das N-terminale Ende der Aminosäure unsubstituiert oder durch eine $-C-(O)-R_2$-Gruppe substituiert ist, worin $R_2$ eine mono- oder polyzyklische $C_6$-$C_{14}$-Arylalkylgruppe; eine mono- oder polyzyklische $C_5$-$C_{14}$-Heteroarylalkylgruppe, die ein oder mehrere identische oder unterschiedliche Heretoatome umfassen kann; eine mono- oder polyzyklische $C_6$-$C_{14}$-Arylalkyloxygruppe oder eine mono- oder polyzyklische $C_5$-$C_{14}$-Heteroarylalkyloxygruppe, die ein oder mehrere identische oder unterschiedliche Heretoatome umfassen kann, ist,
eine $-C(O)-NH-R_3$- oder $-C(S)-NH-R_3$-Gruppierung, worin $R_3$ Wasserstoff; eine lineare oder verzweigte $C_1$-$C_{12}$-Alkylgruppe, unsubstituiert oder durch eine Gruppierung substituiert, die aus OR, NRR', NHR und SR ausgewählt ist, worin R und R' unabhängig voneinander für Wasserstoff, ein lineares $C_1$-$C_{12}$-Alkyl oder ein unsubstituiertes Aryl stehen; eine Arylgruppe, unsubstituiert oder durch eine Gruppierung substituiert, die aus OR, NRR', NHR und SR, wie oben definiert, ausgewählt ist; eine Acylgruppe; eine Formylgruppe; eine Sulfonylgruppe; eine Sulfinylgruppe; eine Silylgruppe, unsubstituiert oder substituiert, unsubstituiert oder durch eine mono- oder polyzyklische $C_6$-$C_{14}$-Arylgruppe substituiert, unsubstituiert oder durch mindestens ein lineares oder verzweigtes $C_1C_6$-Alkyl substituiert; eine Allylgruppe; ein Zuckerrest; eine $-C(O)-OR_4$-Gruppe, worin $R_4$ eine lineares oder verzweigtes $C_1$-$C_{12}$-Alkyl, unsubstituiert oder durch eine Gruppierung substituiert, die aus OR, NRR', NHR und SR, wie oben definiert, ausgewählt ist; eine Amidgruppe; eine Thioamidgruppe; eine Sulfonamidgruppe ist,
eine $-C(O)-R_5$-Gruppierung, worin $R_5$ ein gesättigter $C_5$-$C_{14}$-Heterozyklus ist, der 1 oder 2 Heteroatome umfasst, unsubstituiert oder durch mindestens ein lineares oder verzweigtes $C_1$-$C_6$-Alkyl oder eine Gruppierung substituiert ist, die aus OR, NRR', NHR und SR ausgewählt ist, worin R und R' unabhängig voneinander für Wasserstoff, ein $C_1$-$C_{12}$-Alkyl oder unsubstituiertes Aryl stehen,
- B für eine $-C(O)-R_6$-Gruppierung steht, worin $R_6$ Wasserstoff; ein lineares oder verzweigtes $C_1$-$C_{12}$-Alkyl, vorzugsweise $C_1$-$C_6$-Alkyl, unsubstituiert oder durch eine Gruppierung substituiert, die aus OR, NRR', NHR und SR, wie oben definiert, ausgewählt ist; eine Arylgruppe, unsubstituiert oder durch eine Gruppierung substituiert, die aus OR, NRR', NHR und SR, wie oben definiert, ausgewählt ist, ist; oder außerdem $R_6$ für $OR_7$ steht, wobei $R_7$ ein lineares oder verzweigtes $C_1$-$C_{12}$-Alkyl, vorzugsweise $C_1$-$C_6$-Alkyl, ist.

**2.** Verbindung der Formel (I) nach Anspruch 1, worin zumindest eine der folgenden Bedingungen erfüllt ist:

- A steht für eine -(R1)$_n$-Gruppierung, worin $R_1$ ein Aminosäurerest ist und n = 2 ist;
- A steht für eine -($R_1$)$_n$-Gruppierung, worin $R_1$ ein Aminosäurerest ist, n = 2 ist und das N-terminale Ende der Aminosäure durch eine Arylalcoxycarbonylgruppe, insbesondere eine Benzyloxycarbonylgruppe, substituiert ist;
- A steht für ein Alanylradikal, das an ein Glycinylradikal gebunden ist; und gegebenenfalls an seinem N-terminalen Ende durch eine Arylalcoxycarbonylgruppe, insbesondere eine Benzyloxycarbonylgruppe, substituiert ist;
- A steht für ein Methionylradikal, das an ein Glycinylradikal gebunden ist, und gegebenenfalls an seinem N-terminalen Ende durch eine Arylalcoxycarbonylgruppe, insbesondere eine Benzyloxycarbonylgruppe, substituiert ist.

**3.** Verbindung der Formel (I) nach Anspruch 1, worin A für eine -C(O)-$R_5$-Gruppierung steht, worin $R_5$ eine 2,2-Dimethyl-1,3-dioxolan-Gruppe ist.

**4.** Verbindung der Formel (I) nach einem der Ansprüche 1 bis 3, worin B für eine Acylgruppierung, worin die Alkylgruppe eine $C_1$-$C_6$-Alkylgruppe, insbesondere eine Acetylgruppe ist, oder außerdem für eine Alkoxycarbonylgruppierung steht, worin die Alkylgruppe eine $C_1$-$C_6$-Alkylgruppe, insbesondere eine tert-Butoxycarbonylgruppierung ist.

**5.** Verbindung der Formel (I) nach Anspruch 1, **dadurch gekennzeichnet, dass** sie aus den folgenden Verbindungen ausgewählt ist:

- 7-((tert-Butoxycarbonyl)oxyl)-10,13-dimethyl-17-(6-methylheptan-2-yl)-2, 3,4,7,8,9,10,11,12,13,14,15,16,17-tetradecahydro-1H-cyclopenta[a]phenanthren-3-yl 2-(2-(((benzyloxy)carbonyl)amino)-acetamido)propanoat (Molekül 1.a);
- 7-Acetoxy-10,13-dimethyl-17-(6-methylheptan-2-yl)-2,3,4,7,8,9,10,11,12, 13,14,15,16,17-tetradecahydro-1H-cyclopenta[a]phenanthren-3-yl 2-(2-(((Benzyloxy)carbonyl)amino)-acetamido)propanoat (Molekül 1.b);
- 7-((tert-Butoxycarbonyl)oxy)-10,13-dimethyl-17-(6-methylheptan-2-yl)-2, 3,4,7,8,9,10,11,12,13,14,15,16,17-tetradecahydro-1H-cyclopenta[a]phenanthren-3-yl 2,2-dimethyl-1,3-dioxolan-4-carboxylat (Molekül 2.a);
- 7-Acetoxy-10,13-dimethyl-17-(6-methylheptan-2-yl)-2,3,4,7,8,9,10,11,12, 13,14,15,16,17-tetradecahydro-1H-cyclopenta[a]-phenanthren-3-yl 2,2-dimethyl-1,3-dioxolan-4-carboxylat (Molekül 2.b).

**6.** Verfahren zur Herstellung einer Verbindung der Formel (I) nach einem der Ansprüche 1 bis 5, **dadurch gekennzeichnet, dass** es die folgenden Schritte umfasst:

- Schutz der Hydroxylfunktion in Position 3 des Cholesterols durch eine Schutzgruppierung,
- Einführung einer Ketonfunktion in Position 7,
- Reduzierung der Ketonfunktion in eine Hydroxylfunktion,
- Einführung einer Schutzgruppierung der Hydroxylfunktion in Position 7, entsprechend der Gruppierung B, und
- Entschützen der Hydroxylfunktion in Position 3.

**7.** Verfahren nach Anspruch 6, **dadurch gekennzeichnet, dass** nach dem Entschützen die Hydroxylfunktion in Position 3 durch die gewünschte Gruppierung A substituiert werden kann.

**8.** Pharmazeutische Zusammensetzung, umfassend mindestens eine Verbindung der Formel (I) nach einem der Ansprüche 1 bis 5 und einen pharmazeutisch verträglichen Träger.

**9.** Pharmazeutische Zusammensetzung nach Anspruch 8, **dadurch gekennzeichnet, dass** die Verbindung der Formel (I) als einziger Wirkstoff oder in Kombination mit einem Antikrebsmittel verwendet wird.

**10.** Pharmazeutische Zusammensetzung nach einem der Ansprüche 8 oder 9, **dadurch gekennzeichnet, dass** sie aus einem Liposom oder aus einer alkoholischen Lösung von mindestens einer Verbindung der Formel (I) allein oder in Kombination mit einem anderen Wirkstoff besteht.

**11.** Verbindung der Formel (I) nach einem der Ansprüche 1 bis 5 zur Verwendung bei der Behandlung von Erkrankungen, die transformierte Astrozytenzellen mit einschließen.

**12.** Verbindung der Formel (I) nach einem der Ansprüche 1 bis 5 zur Verwendung bei der Behandlung von multiplem Glioblastom.

**13.** Verbindung der Formel (I) nach einem der Ansprüche 1 bis 5 zur Verwendung bei der Behandlung von Erkrankungen, die transformierte Astrozytenzellen mit einschließen, wobei die Behandlung eine aufeinanderfolgende Behandlung mit mindestens einem Schritt einer Verabreichung einer ersten Verbindung der Formel (I) und mindestens einem Schritt einer Verabreichung einer zweiten Verbindung der Formel (I), die sich von der ersten unterscheidet, ist.

**14.** Verbindung der Formel (I) nach einem der Ansprüche 1 bis 5 zur Verwendung bei der Behandlung von multiplem Glioblastom, wobei die Behandlung eine aufeinanderfolgende Behandlung mit mindestens einem Schritt einer Verabreichung einer ersten Verbindung der Formel (I) und mindestens einem Schritt einer Verabreichung einer zweiten Verbindung der Formel (I), die sich von der ersten unterscheidet, ist.

**15.** Pharmazeutische Zusammensetzung nach einem der Ansprüche 8 bis 10, **dadurch gekennzeichnet, dass** die Verbindung der Formel (I) 7-Acetoxy-10,13-dimethyl-17-(6-methylheptan-2-yl)-2,3,4,7,8,9,10,11,12, 13,14,15,16,17-tetradecahydro-1H-cyclopenta[a]-phenanthren-3-yl 2,2-dimethyl-1,3-dioxolan-4-carboxylat (Molekül 2.b) ist.

**16.** Verbindung der Formel (I) nach Anspruch 5 zur Verwendung nach Anspruch 14, **dadurch gekennzeichnet, dass** die erste Verbindung der Formel (I) 7-((tert-Butoxycarbonyl)oxyl)-10,13-dimethyl-17-(6-methylheptan-2-yl)-2, 3,4,7,8,9,10,11,12,13,14,15,16,17-tetradecahydro-1H-cyclopenta[a]phenanthren-3-yl 2-(2-(((benzyloxy)carbonyl)amino)-acetamido)propanoat (Molekül 1.a) ist und die zweite Verbindung der Formel (I) 7-((tert-Butoxycarbonyl)-oxy)-10,13-dimethyl-17-(6-methylheptan-2-yl)-2,3,4,7,8,9,10,11,12,13,14, 15,16,17-tetradecahydro-1H-cyclopenta[a]phenanthren-3-yl 2,2-dimethyl-1,3-dioxolan-4-carboxylat (Molekül 2.a) ist.

## Claims

**1.** Compound of formula (I)

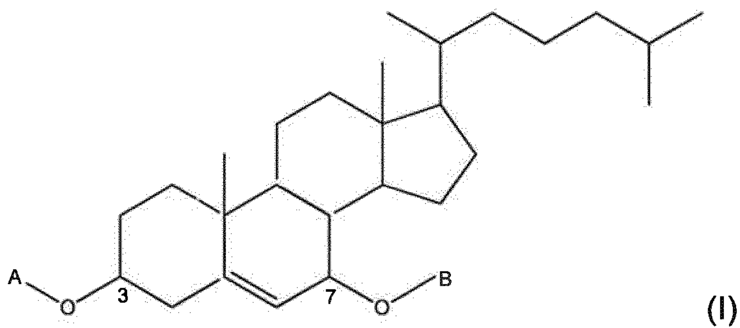

(I)

in which

- A represents

an -$(R_1)_n$- group in which $R_1$ is an amino acid residue bound by its C-terminal end and n = 1 to 3, each $R_1$ being identical or different, in which the N-terminal end of said amino acid is unsubstituted or substituted with a -C-(O)-$R_2$ group in which $R_2$ is a mono- or polycyclic $C_6$-$C_{14}$ arylalkyl group; a mono- or polycyclic $C_5$-$C_{14}$ heteroarylalkyl group that can comprise one or more heteroatoms, which may be identical or different; a mono- or polycyclic $C_6$-$C_{14}$ arylalkyloxy group or a mono- or polycyclic $C_5$-$C_{14}$ heteroarylalkyloxy group that can comprise one or more heteroatoms, which may be identical or different,

a -C(O)-NH-$R_3$ or -C(S)-NH-$R_3$ group in which $R_3$ is hydrogen; a $C_1$-$C_{12}$ alkyl group, linear or branched, unsubstituted or substituted with a group selected from OR, NRR', NHR and SR, where R and R' independently represent hydrogen, a linear $C_1$-$C_{12}$ alkyl or an unsubstituted aryl; an aryl group unsubstituted or substituted with a group selected from OR, NRR', NHR and SR as defined above; an acyl group; a formyl group; a sulphonyl group; a sulphinyl group, a silyl group unsubstituted or substituted unsubstituted or substituted with a mono- or polycyclic $C_6$-$C_{14}$ aryl group, unsubstituted or substituted with at least one linear or branched $C_1$-$C_6$ alkyl; an allyl group; a sugar residue; a -C(O)-$OR_4$ group in which $R_4$ is a linear or branched $C_1$-$C_{12}$ alkyl unsubstituted or substituted with a group selected from OR, NRR', NHR and SR, as defined above; an amide group;a thioamide group; a sulphonamide group,

a -C(O)-$R_5$ group in which $R_5$ is a saturated $C_5$-$C_{14}$ heterocycle comprising 1 or 2 heteroatoms, unsubstituted

or substituted with at least one linear or branched $C_1$-$C_6$ alkyl or a group selected from OR, NRR', NHR and SR, where R and R' independently represent hydrogen, a $C_1$-$C_{12}$ alkyl or an unsubstituted aryl.
- B represents a -C(O)-$R_6$ group in which $R_6$ is hydrogen; a $C_1$-$C_{12}$, preferably $C_1$-$C_6$, alkyl, linear or branched, unsubstituted or substituted with a group selected from OR, NRR', NHR and SR, as defined above; an aryl group, unsubstituted or substituted by a group selected from OR, NRR', NHR and SR, as defined above; or else $R_6$ represents $OR_7$, in which $R_7$ is a linear or branched $C_1$-$C_{12}$, preferably $C_1$-$C_6$, alkyl.

**2.** Compound of formula (I) according to claim 1, in which at least one of the following conditions is fulfilled:

- A represents an -$(R_1)_n$- group in which $R_1$ is an amino acid residue and n = 2;
- A represents an -$(R_1)_n$- group in which $R_1$ is an amino acid residue, n = 2 and the N-terminal end of said amino acid is substituted with an arylalkoxycarbonyl group, in particular benzyloxycarbonyl;
- A represents an alanyl radical linked to a glycinyl radical, optionally substituted on its N-terminal end with an arylalkoxycarbonyl group, in particular benzyloxycarbonyl;
- A represents a methionyl radical linked to a glycinyl radical, optionally substituted on its N-terminal end with an arylalkoxycarbonyl group, in particular benzyloxycarbonyl.

**3.** Compound of formula (I) according to claim 1, in which A represents a -C(O)-$R_5$ group in which $R_5$ is a 2,2-dimethyl-1,3-dioxolane group.

**4.** Compound of formula (I) according to one of claims 1 to 3, in which B represents an acyl group in which the alkyl group is $C_1$-$C_6$, in particular acetyl, or an alkoxycarbonyl group in which the alkyl group is $C_1$-$C_6$, in particular a *tert*-butoxycarbonyl group.

**5.** Compound of formula (I), **characterized in that** it is selected from the following compounds:

- 7-((*tert*-butoxycarbonyl)oxy)-10,13-dimethyl-17-(6-methylheptan-2-yl)-2,3,4,7,8,9,10,11,12,13,14,15,16,17-tetradecahydro-1*H*-cyclopenta[*a*]phenanthren-3-yl 2-(2-(((benzyloxy)carbonyl)amino)-acetamido)propanoate (molecule 1.a);
- 7-acetoxy-10,13-dimethyl-17-(6-methylheptan-2-yl)-2,3,4,7,8,9,10,11,12,13,14,15,16,17-tetradecahydro-1H-cyclopenta[a]phenanthren-3-yl 2-(2-(((benzyloxy)carbonyl)amino)-acetamido)propanoate (molecule 1.b);
- 7-((tert-butoxycarbonyl)oxy)-10,13-dimethyl-17-(6-methylheptan-2-yl)-2,3,4,7,8,9,10,11,12,13,14,15,16,17-tetradecahydro-1H-cyclopenta[a]phenanthren-3-yl 2,2-dimethyl-1,3-dioxolane-4-carboxylate (molecule 2.a);
- 7-acetoxy-10,13-dimethyl-17-(6-methylheptan-2-yl)-2,3,4,7,8,9,10,11,12,13,14,15,16,17-tetradecahydro-1 H-cyclopenta[a]-phenanthren-3-yl 2,2-dimethyl-1,3-dioxolane-4-carboxylate (molecule 2.b).

**6.** Method for the preparation of a compound of formula (I) according to any one of claims 1 to 5, **characterized in that** it comprises the following steps:

- protection of the hydroxyl function in position 3 of the cholesterol with a protective group,
- introduction of a ketone function in position 7,
- reduction of the ketone function to a hydroxyl function,
- introduction of a protective group on the hydroxyl function in position 7, corresponding to the B group, and
- deprotection of the hydroxyl function in position 3.

**7.** Method according to claim 6, **characterized in that**, after deprotection, the hydroxyl function in position 3 can be substituted with the desired A group.

**8.** Pharmaceutical composition comprising at least one compound of formula (I) according to any one of claims 1 to 5 and a pharmaceutically acceptable vehicle.

**9.** Pharmaceutical composition according to claim 8, **characterized in that** said compound of formula (I) is used as the only active ingredient, or in combination with an anti-cancer agent.

**10.** Pharmaceutical composition according to any one of claims 8 or 9, **characterized in that** it consists of a liposome or an alcohol solution of at least one compound of formula (I), alone or in a mixture with another active ingredient.

**11.** Compound of formula (I) according to any one of claims 1 to 5, for use in the treatment of diseases involving

transformed astrocyte cells.

**12.** Compound of formula (I) according to any one of claims 1 to 5, for use in the treatment of glioblastoma multiforme.

**13.** Compound of formula (I) according to any one of claims 1 to 5, for use in the treatment of diseases involving transformed astrocyte cells, said treatment being a sequential treatment comprising at least one step of administering a first compound of formula (I) and at least one step of administering a second compound of formula (I), different from the first.

**14.** Compound of formula (I) according to any one of claims 1 to 5, for use in the treatment of glioblastoma multiforme, said treatment being a sequential treatment comprising at least one step of administering a first compound of formula (I) and at least one step of administering a second compound of formula (I), different from the first.

**15.** Pharmaceutical composition according to any one of claims 8 to 10, **characterized in that** said compound of formula (I) is the 7-acetoxy-10,13-dimethyl-17-(6-methylheptan-2-yl)-2,3,4,7,8,9,10,11,12,13,14,15,16,17-tetradecahydro-1H-cyclopenta[a]-phenanthren-3-yl 2,2-dimethyl-1,3-dioxolane-4-carboxylate (molecule 2.b).

**16.** Compound of formula (I) according to claim 5 , for use according to claim 14, **characterized in that** the first compound of formula (I) is the 7-((*tert*-butoxycarbonyl)oxyl)-10,13-dimethyl-17-(6-methylheptan-2-yl)-2,3,4,7,8,9,10,11,12,13,14,15,16,17-tetradecahydro-1*H*-cyclopenta[a]phenanthren-3-yl 2-(2-(((benzyloxy)carbonyl)amino)-acetamido)propanoate (molecule 1.a) and the second compound of formula (I) is the 7-((tert-butoxycarbonyl)oxy)-10,13-dimethyl-17-(6-methylheptan-2-yl)-2,3,4,7,8,9,10,11,12,13,14,15,16,17-tetradecahydro-1H-cyclopenta[a]phenanthren-3-yl 2,2-dimethyl-1,3-dioxolane-4-carboxylate (molecule 2.a).

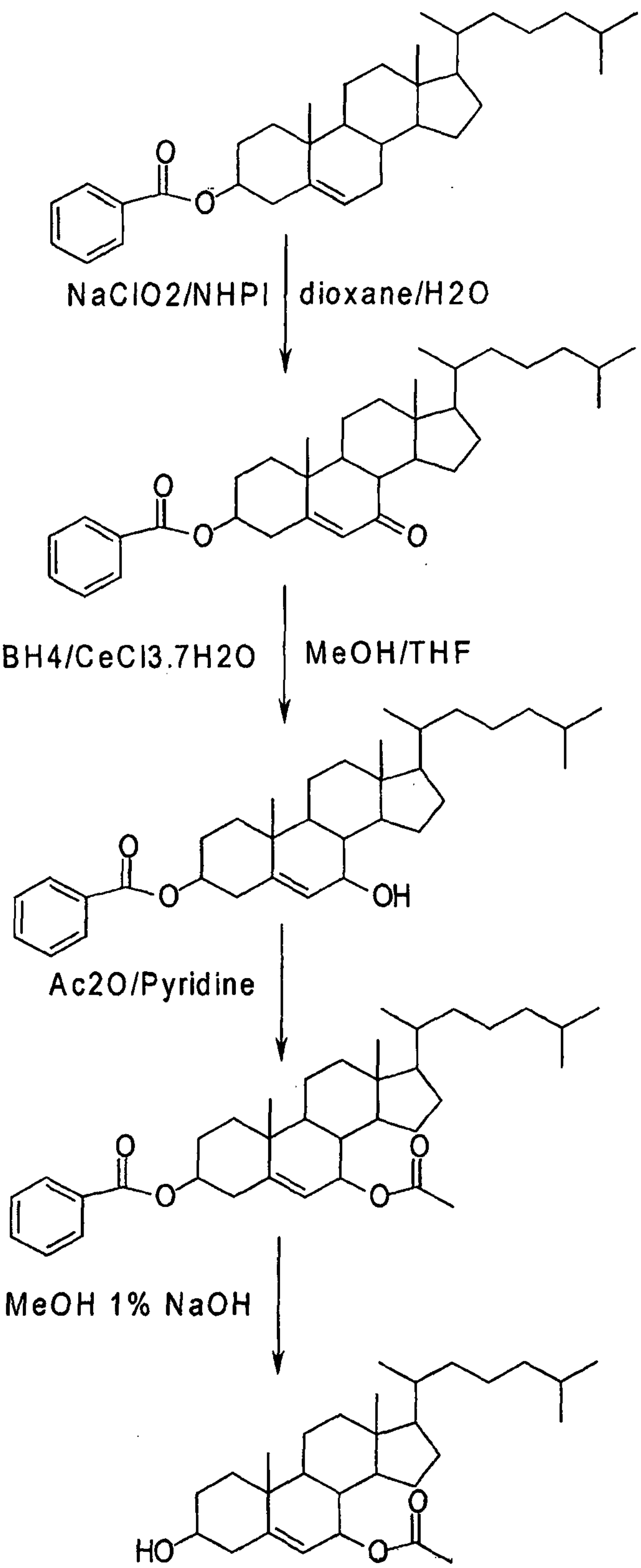


1.0

1.1

1.2

1.3

1.4

Fig. 1

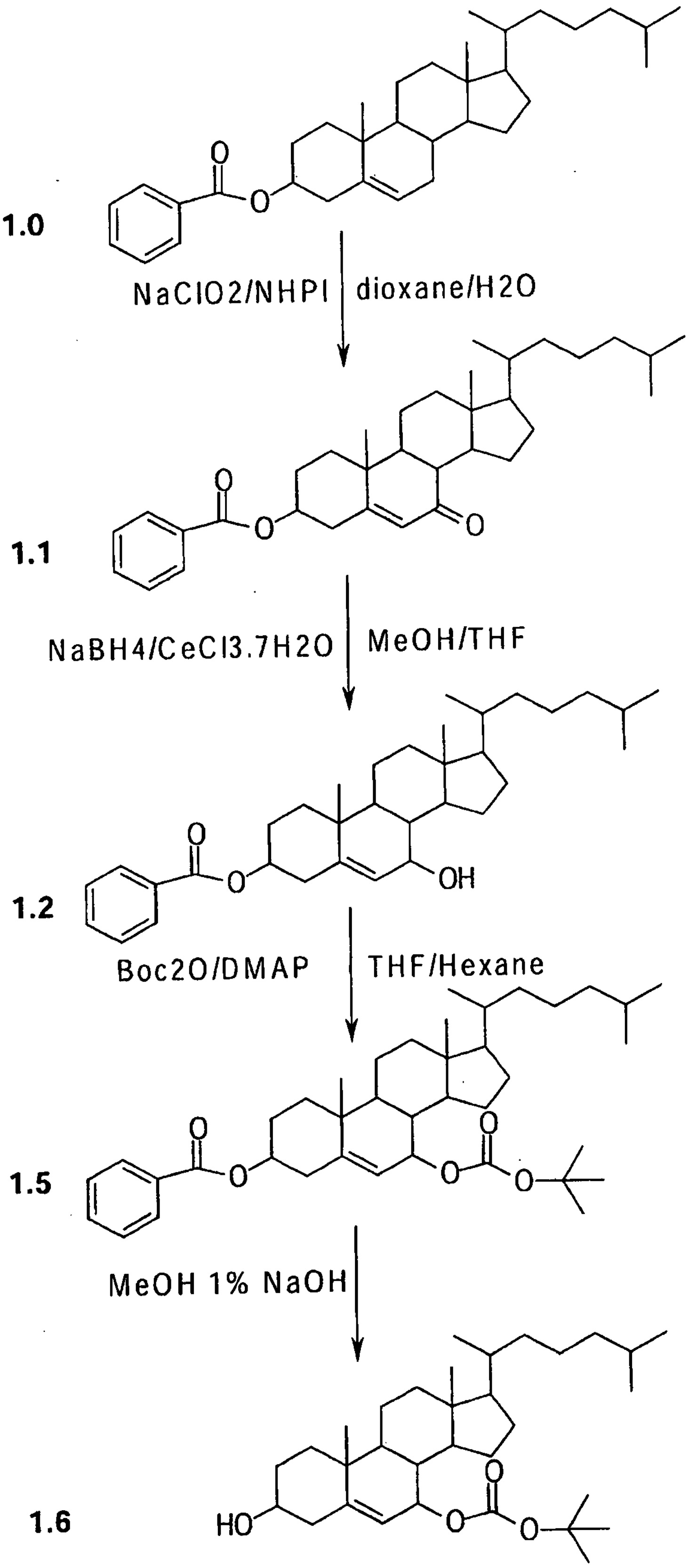


1.0

1.1

1.2

1.5

1.6

Fig. 2

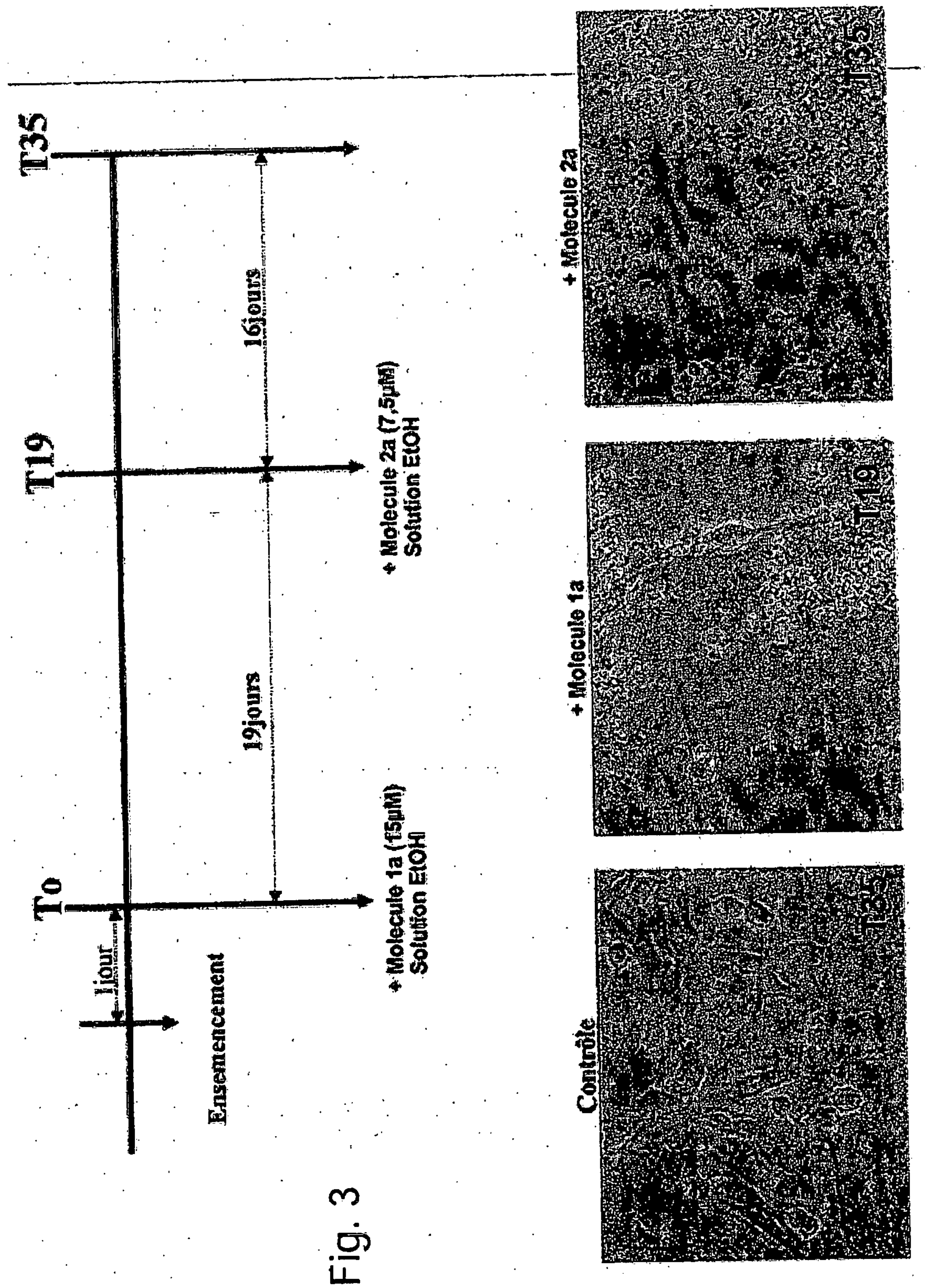
T35
T19
To
1jour
Ensemencement
19jours
16jours
+ Molecule 1a (15µM)
Solution EtOH
+ Molecule 2a (7,5µM)
Solution EtOH
Contrôle
T35
+ Molecule 1a
T19
+ Molecule 2a
T35

Fig. 3

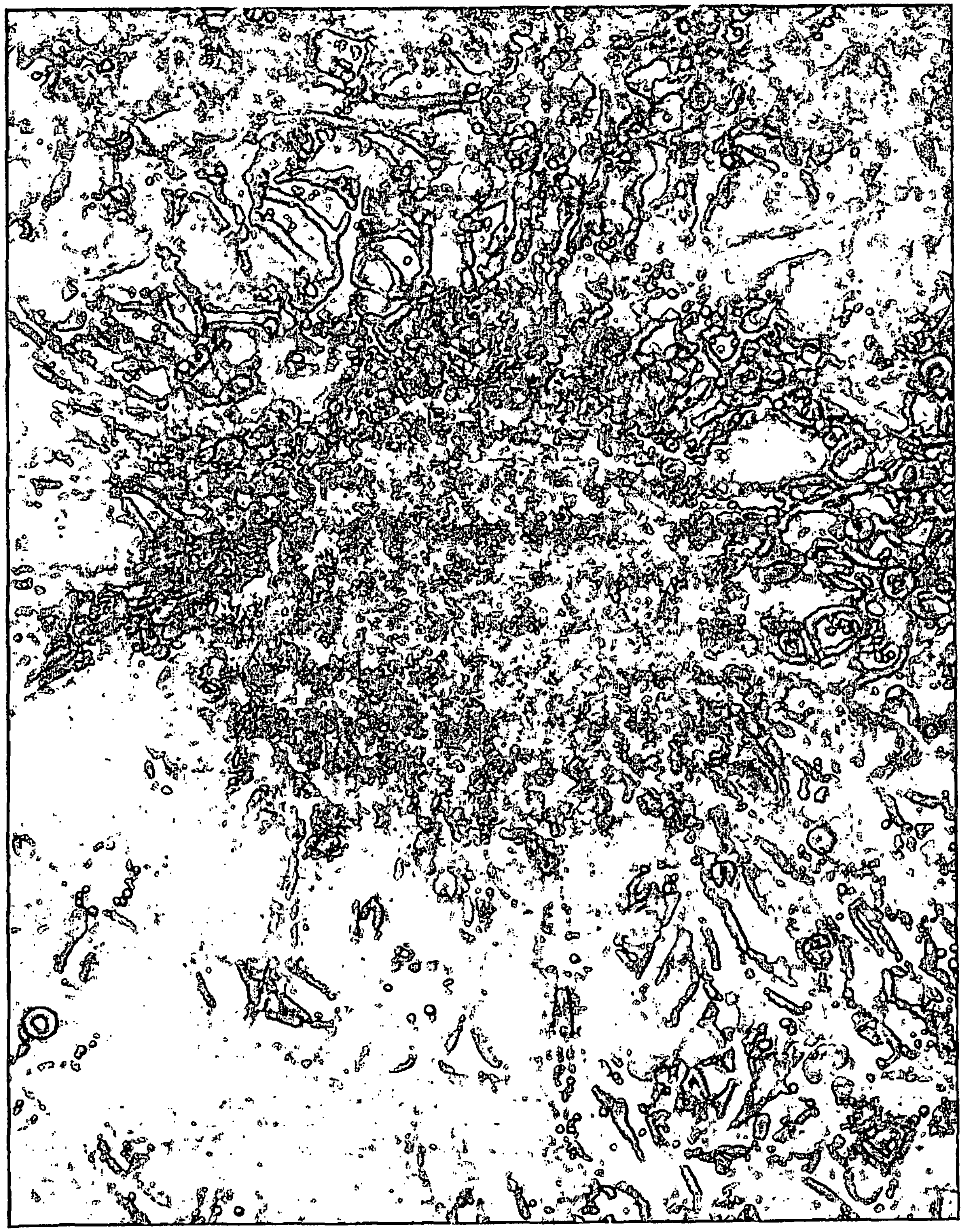

Fig. 4

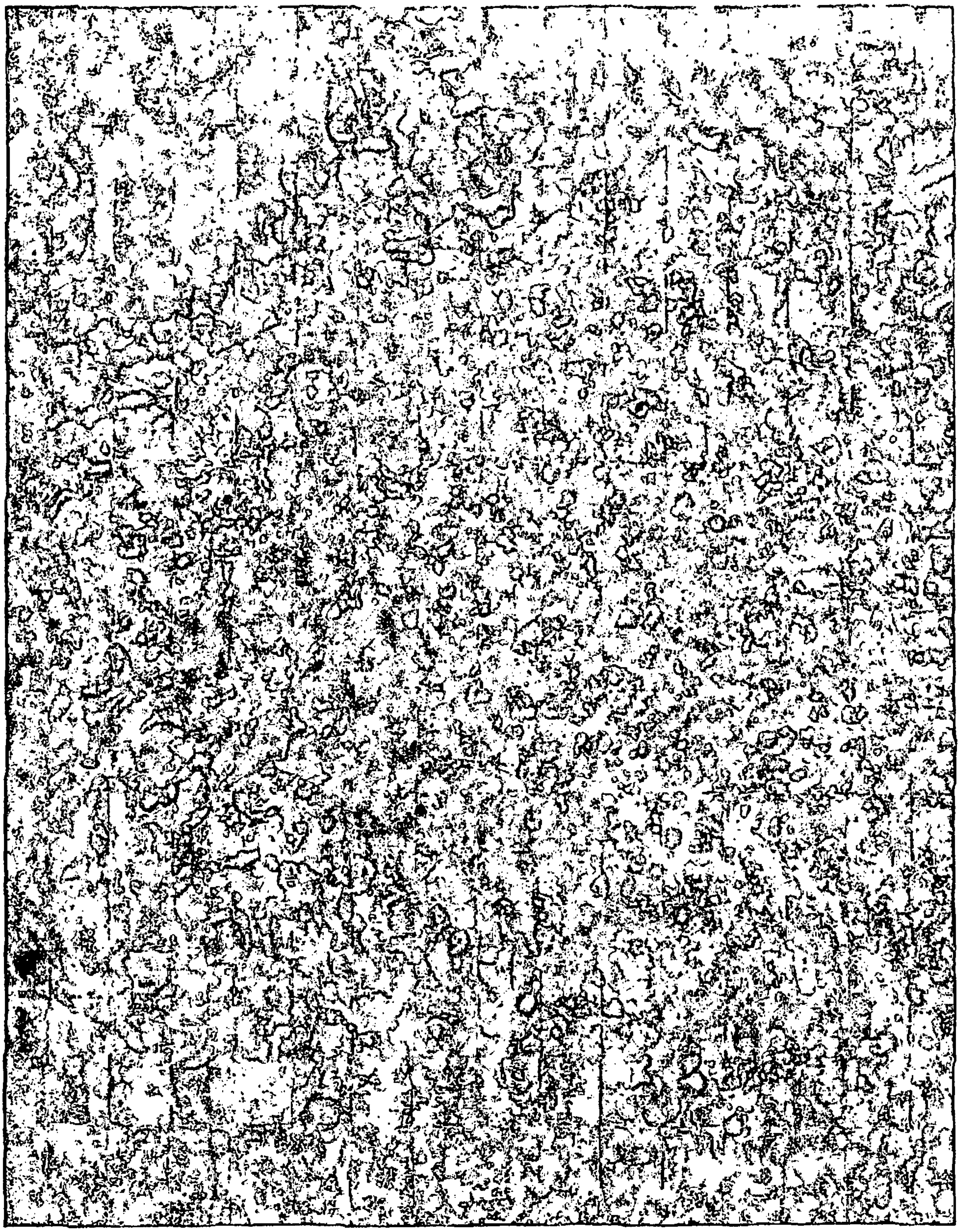

Fig. 5

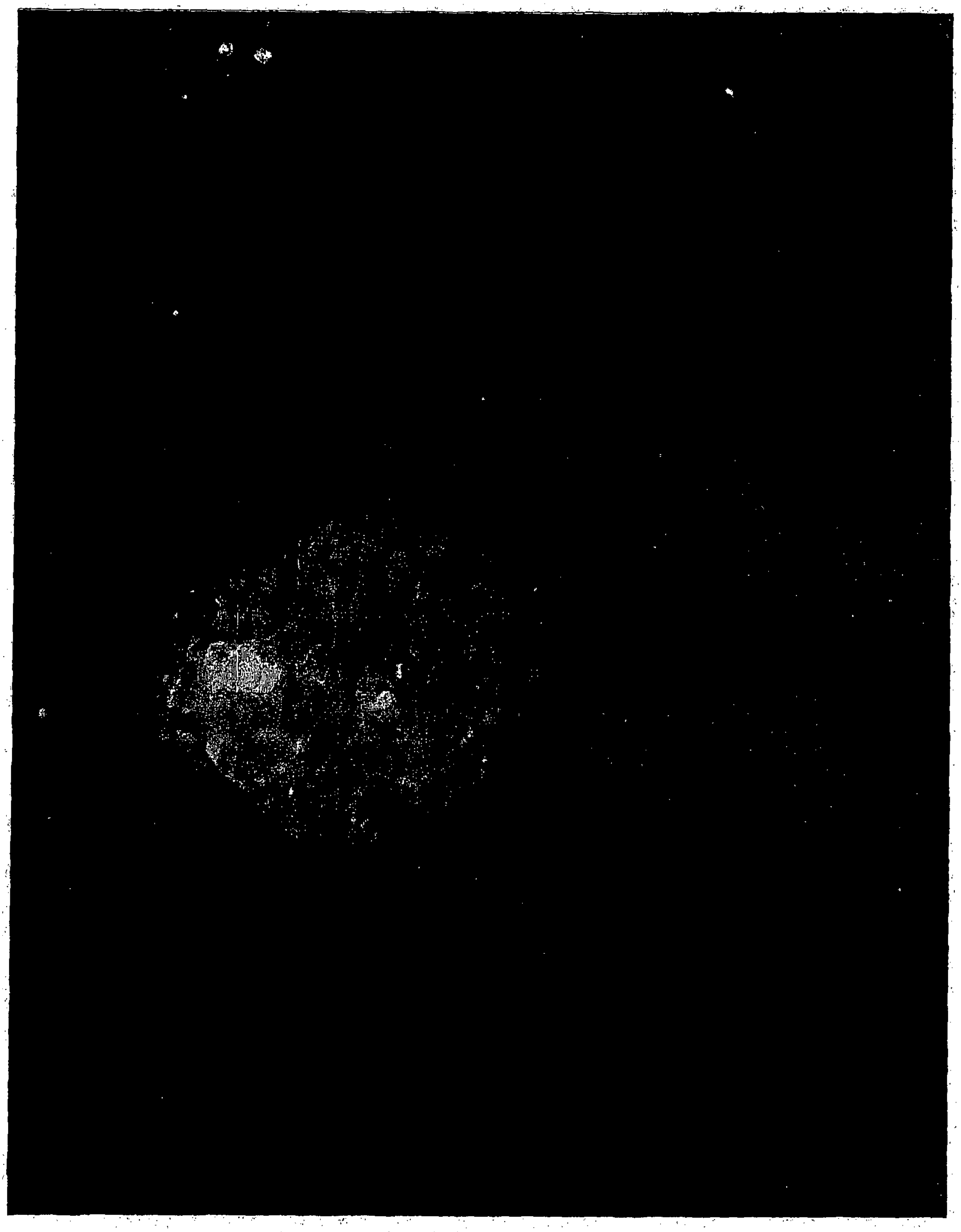

Fig. 6

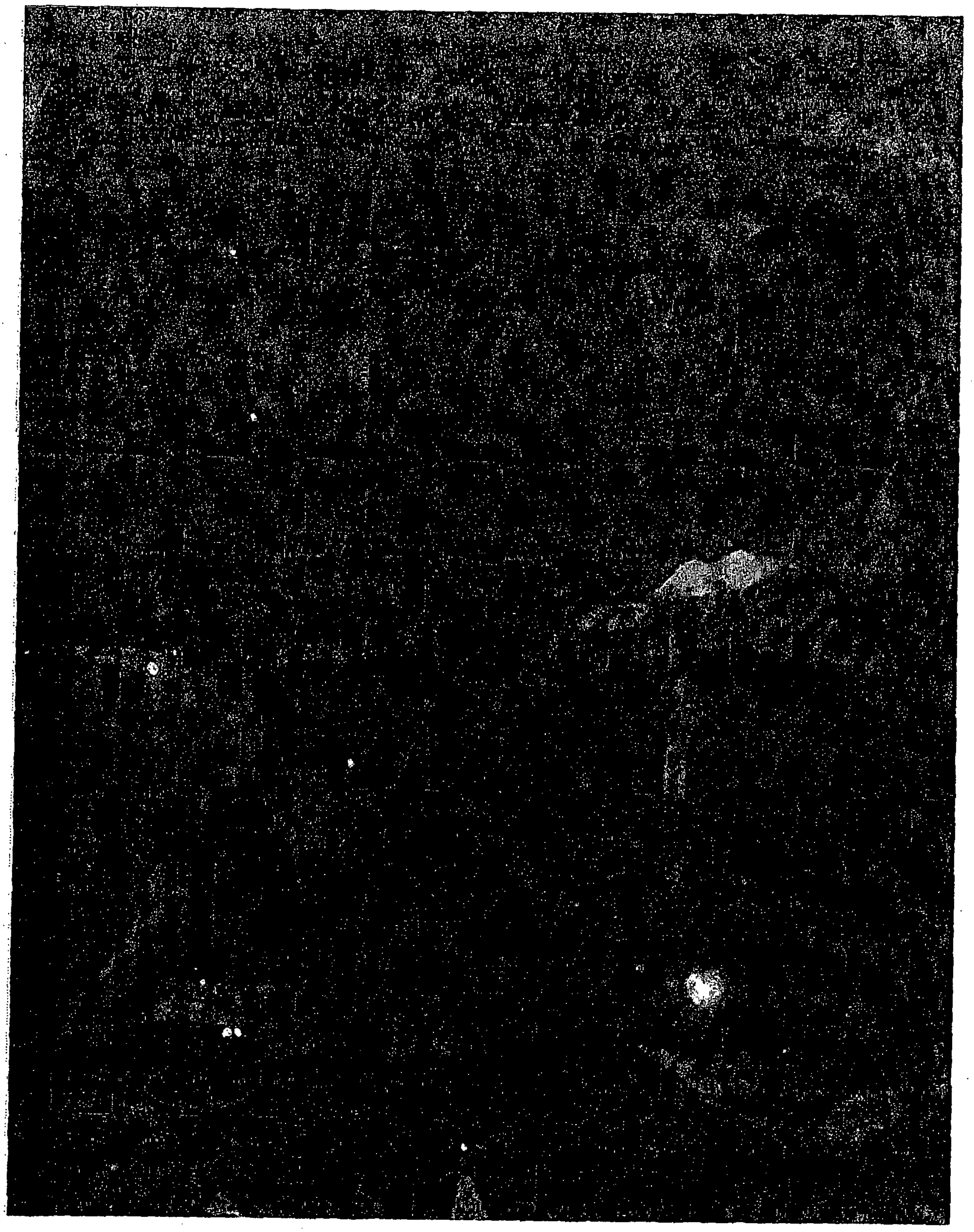

Fig. 7

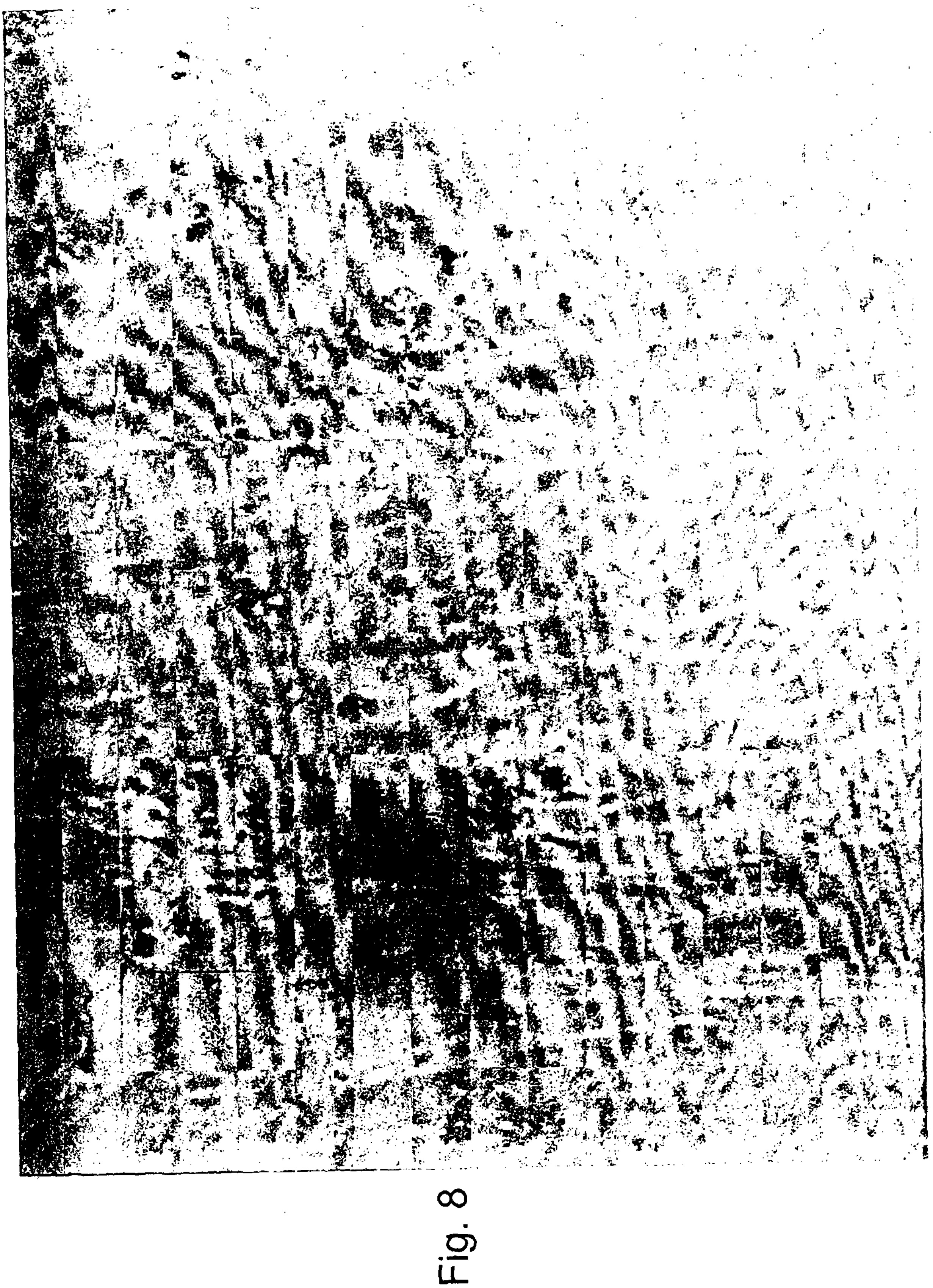

Fig. 8

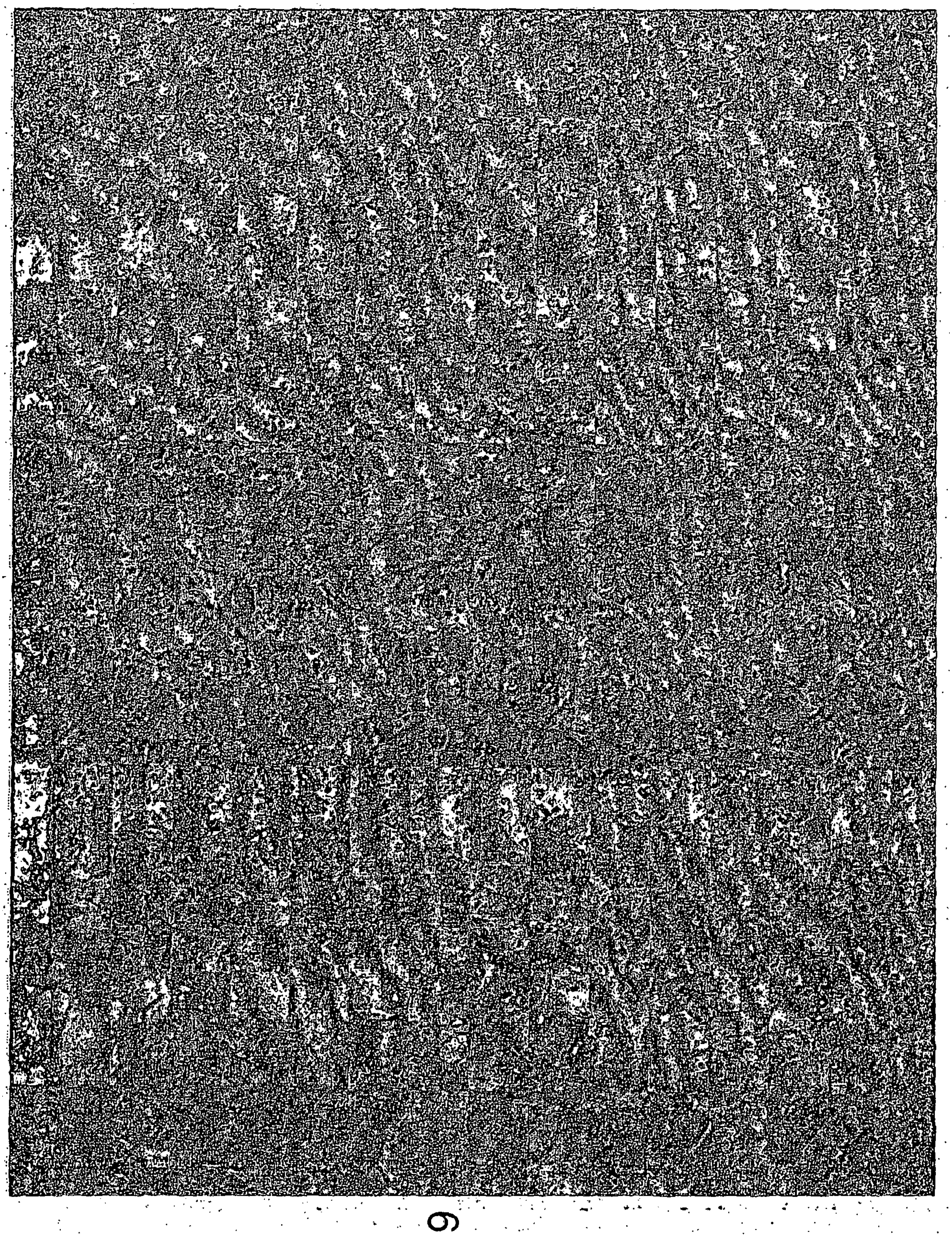

Fig. 9

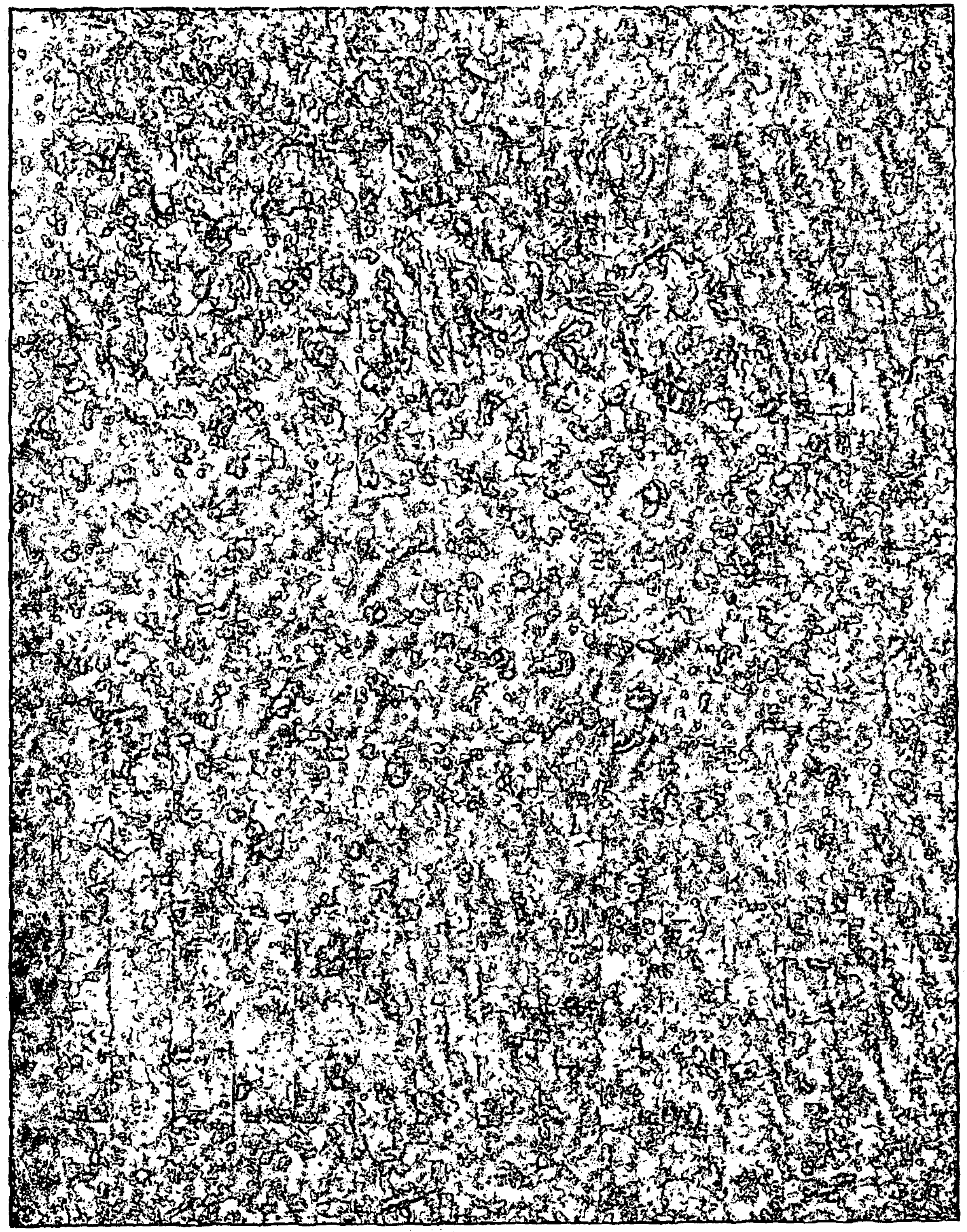

Fig. 10

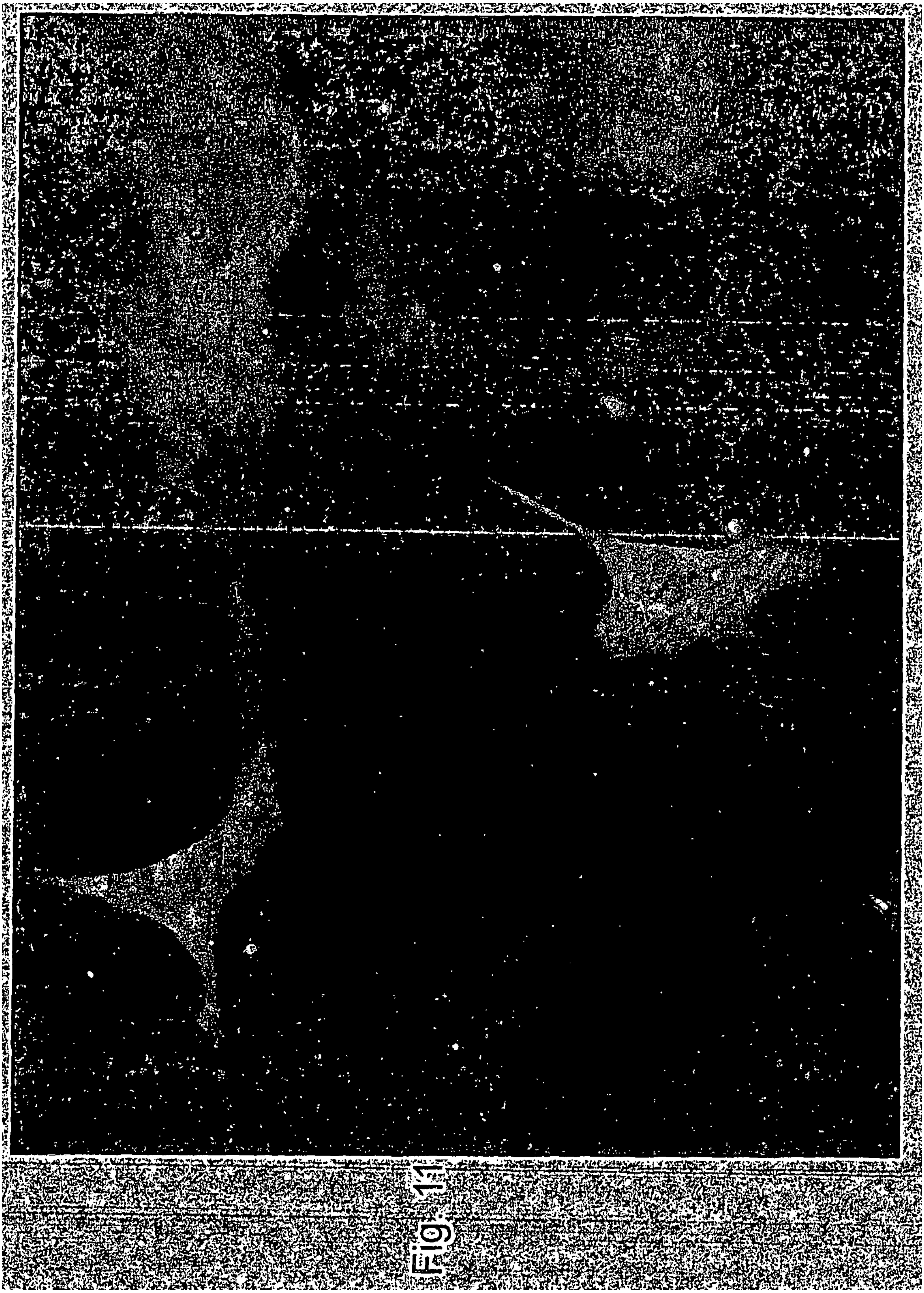

Fig. 11

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Littérature non-brevet citée dans la description


- Medical Application of Liposomes. Scientific Societies Press, 1986
- **KANDO T ; SETOGUCHI T ; TAGA T.** *PNAS,* 2004, vol. 101, 781-786
- **ZHOU Y ; ZHOU Y ; SHINGU T ; FENG L ; CHEN Z ; OGASAWARA M ; KEATING MJ.** *J. Biol. Chem.,* 2011, vol. 286, 32843-32853
- **WARBURG O.** *Biochemische Zeitscrift,* 1923, vol. 142, 317-333
- **TENNANT DA ; DURAN RV ; GOTTLIEB E.** *Nature Reviews Cancer.,* 2010, vol. 10, 267-277
- **CHENG KP ; NAGANO H ; BANG L ; OURISSON G ; BECK JP.** *Journal of Chemistry Research (M),* 1977, vol. 217, 2501-2521
- **CARVALHO JFS ; CRUZ SILVA MM ; MOREIRA JN ; SIMOES S ; SA E MELO ML.** *Journal of Médicinal Chemistry,* 2011, vol. 54, 6375-6393
- **KUPFERBERG A ; TELLER G ; BEHR P ; LERAY C ; URBAN PF ; VINCENDON G ; MERSEL M.** *Biochim Biophys Acta,* 1989, vol. 1013, 231-23
- **KUPFERBERG A ; BEHR P ; MERSEL M.** *Biochim Biophys Acta,* 1990, vol. 1046, 106-109
- **ADAMCZYCK M ; SCHERRER E ; KUPFERBERG A ; MALVIYA AN ; MERSEL M.** *Journal of Neuroscience Research,* 1988, vol. 53, 38-50
- **WERTHLE M ; BOCHELEN D ; ADAMCZYCK M ; KUPFERBERG A ; POULET P ; CHAMBRON J ; LUTZ P ; PRIVATA ; MERSEL M.** *Cancer Research,* 1994, vol. 54, 998-1003
- **CLARION L ; SCHINDLER M ; DE WEILLE J ; LOLMÈDE K ; LAROCHE-CLARY A ; URO-COSTE E ; ROBERT J ; MERSEL M ; BAKALARA N.** *Biochemical Pharmacology,* 2012, vol. 83, 37-46
- **MANFORD K ; PATTERSON JR.** *Methods in Enzymology,* 1979, vol. 58, 150
- **BENDA P ; LIGHTBODY J ; SATO G ; LEVINE L ; SWEET W.** *Science,* 1968, vol. 161, 370-371
- **RAKOTOARIVELO C ; ADAMCZYCK M ; DESGEORGES M ; LANGLEY K ; LORENTZ JG ; MANN A ; RICARD D ; SCHERRER E ; PRIVAT A ; MERSEL M.** *Anticancer Research.,* 2006, vol. 26, 2053-2062
- **FOLCH J ; LEES M ; SLOANE-STANLEY GH.** *Journal of Biological Chemistry,* 1957, vol. 226, 497-509
- **SENSENBRENNER M ; DEVILLIERS G ; BOCK E ; PORTE A.** *Differentiation,* 1980, vol. 17, 51-61